# FASCICULE DE BREVET EUROPEEN

(11) **EP 1 827 396 B1**
(45) Date de publication et mention de la délivrance du brevet: **01.06.2016**
(21) Numéro de dépôt: 05819012.5
(22) Date de dépôt: 02.11.2005
(51) Int. Cl.: A61K 9/52

(54) **MEDICAMENT ORAL A LIBERATION MODIFIEE D'AU MOINS UN PRINCIPE ACTIF SOUS FORME MULTIMICROCAPSULAIRE**
ORALES MEDIKAMENT FÜR DIE MODIFIZIERTE FREISETZUNG VON MINDESTENS EINEM WIRKSTOFF IN MULTIMIKROKAPSELFORM
ORAL MEDICAMENT FOR THE MODIFIED RELEASE OF AT LEAST ONE ACTIVE PRINCIPLE, IN MULTIMICROCAPSULE FORM

(30) Priorité: 24.11.2004 FR 0452748
(43) Date de publication de la demande: 05.09.2007
(73) Titulaire: Flamel Ireland Limited, Dublin 2 (IE)
(72) Inventeur: GUIMBERTEAU, Florence, F-33450 MONTUSSAN (FR); CASTAN, Catherine, F-69530 ORLIENAS (FR); MEYRUEIX, Rémi, F-69009 LYON (FR); SOULA, Gérard, F-69330 Meyzieu (FR)
(74) Mandataire: Cabinet Plasseraud
(86) Numéro de dépôt international: PCT/FR2005/050922
(87) Numéro de publication internationale: WO 2006/056711

(56) Documents cités:
- EP-A- 1 101 490
- US-A- 4 434 153
- US-A- 4 844 905

## Description

### Domaine de l'invention

Le domaine de la présente invention est celui des systèmes microparticulaires à libération retardée et contrôlée de principe(s) actif(s) PA, destinés à une administration par voie orale.

Les PA envisagés dans la présente invention sont en particulier ceux qui ont une absorption essentiellement limitée aux parties hautes du tractus gastro-intestinal, situées en amont du colon (de la jonction iléo-cæcale), et qui représentent une grande majorité des principes actifs pharmaceutiques. Les principes actifs plus spécialement visés sont des principes actifs "peu solubles".

Plus précisément, l'invention se rapporte à une forme galénique microparticulaire à libération retardée et contrôlée pour laquelle la phase de libération contrôlée est déclenchée de façon certaine grâce à un double mécanisme : libération "temps dépendant" déclenchée au bout d'une certaine durée de séjour dans l'estomac et libération "pH dépendant" déclenchée par un changement de pH lors de l'entrée des particules dans le petit intestin et qui débute sans temps de latence. Les microparticules de la présente invention sont des microcapsules contenant au moins un Principe Actif (PA) -à l'exclusion du carvédilol -, de granulométrie inférieure à 2000 microns individuellement recouvertes par une pellicule d'enrobage permettant la libération retardée et contrôlée du PA.

L'invention a également trait aux microcapsules à libération modifiée d'au moins un principe actif, prises en tant que telles.

Dans le présent exposé, l'expression "principe actif peu soluble", désigne tout principe actif à l'exclusion du carvédilol, ayant une solubilité inférieure ou égale à environ 50 g/l, de préférence inférieure ou égale à environ 20g/l, plus préférentiellement encore inférieure ou égale à environ 5g/l, et par exemple inférieure ou égale à 0,1 g/l.

Dans le présent exposé, le terme "microcapsules" désigne des microparticules de principe actif pelliculées par au moins un revêtement permettant la libération modifiée d'au moins un principe actif (en particulier un principe actif peu soluble).

Dans le présent exposé, le terme "carvédilol" désigne le carvédilol per se, un ou plusieurs sels de carvédilol pharmaceutiquement acceptables ou un ou plusieurs esters de carvédilol pharmaceutiquement acceptables ou tout mélange de ces actifs.

Dans le présent exposé, l'expression "libération modifiée", désigne indifféremment une libération prolongée du (ou des) principe(s) actif(s), débutant dès 1a mise en contact des microcapsules avec le milieu de dissolution et s'étendant de 0,5h à 24h, de préférence de 1 à 10h, ou une libération du (ou des) principe(s) actif(s) ne débutant qu'après une durée prédéterminée (temps de latence) allant par exemple de 0,5 à plusieurs heures, avec un temps de libération de 50 % du (ou des) principe(s) actif(s), qui est typiquement de plusieurs heures et qui peut s'étendre de 0,5 à 30 heures, par exemple.

Dans le présent exposé, l'expression "libération immédiate", désigne une libération du (ou des) principe(s) actif(s) débutant dès la mise en contact de la forme galénique avec le milieu de dissolution (in vivo ou in vitro) avec un temps de libération de 80 % du (ou des) principe(s) actif(s) qui est inférieur ou égal à 1 h, et par exemple inférieur ou égal à 20 min.

Les systèmes à libération retardée et contrôlée de principe(s) actif(s) sont particulièrement utiles lorsqu'il est souhaitable, pour des raisons de chronobiologie, que le(les) principe(s) actif(s) soi(en)t "bioabsorbé(s)" à une heure précise de la journée afin d'être en phase avec le cycle circadien. Il peut être, par exemple, avantageux que le(les) principe(s) actif(s) soi(en)t bioabsorbé(s) très tôt le matin afin d'assurer une couverture thérapeutique au réveil du patient sans pour autant le contraindre à un réveil prématuré. Pour ce faire, le système galénique ingéré par le patient, par exemple le soir après le repas, doit permettre une libération retardée du(des) principe(s) actif(s).

Sachant qu'une autre règle imposée au galéniste est de garantir que le médicament prescrit sera absorbé par le patient, il est crucial, dans le cas d'une forme à libération retardée, d'avoir une totale garantie de libération du principe actif à un instant déterminé de façon à obtenir l'effet thérapeutique. Or, force est de constater que les formes à libération retardée existant jusqu'à une époque récente, ne pouvaient assurer de façon certaine la libération du PA dans un délai prescrit, qui peut être vital pour le patient dans certaines pathologies, comme par exemple celui les maladies cardiovasculaires.

Une autre propriété recherchée pour les systèmes à libération retardée et contrôlée de principe(s) actif(s) est l'amélioration du profil de concentration plasmatique obtenu après administration. Le but visé est d'obtenir un profil plasmatique qui se maintienne au dessus de la concentration thérapeutique efficace, pendant le plus longtemps possible, afin de maximiser la durée d'action du(des) principe(s) actif(s), et donc son(leur) efficacité thérapeutique. Ce but se heurte au temps de résidence du(des) principe(s) actif(s) dans le compartiment sanguin, qui est le plus souvent très inférieur à une journée. Pour atteindre ce but, il conviendrait donc de prolonger le temps de bio-absorption du(des) principe(s) actif(s) PA par un ajustement judicieux de la libération du(des) principe(s) actif(s) devant sa(leur) fenêtre de bio-absorption, dans les parties hautes du tractus gastro-intestinal.

Diverses formes à libération modifiée de principe(s) actif(s) ont été développées, pour tenter de résoudre les problèmes sus évoqués de chronothérapie et de maintien d'un profil plasmatique élevé le plus longtemps possible.

On connaît ainsi des formes à libération retardée dépendante du pH ("pH dépendante"), qui sont obtenues par revêtement du(des) principe(s) actif(s) au moyen d'une couche de polymère entérique, par exemple, de copolymère d'acide méthacrylique et d'ester méthylique d'acide méthacrylique: EUDRAGIT®L. Ce type de revêtement entérique est connu pour présenter une perméabilité réduite dans les conditions de pH acide de l'estomac et se dissoudre lorsque le pH remonte à une valeur proche de celle régnant dans le petit intestin, libérant ainsi le (les) principe(s) actif(s). Cependant, la variabilité intra et interindividuelle des conditions de pH gastrique et de la durée de la vidange gastrique ne permettent pas d'assurer de façon certaine la libération du(des) principe(s) actif(s) après une durée déterminée.

On connaît par ailleurs des systèmes à libération retardée purement dépendante du temps après l'ingestion ("temps dépendant"), c'est à dire des systèmes pour lesquels la libération du(des) principe(s) actif(s)se déclenche au bout d'une durée déterminée de séjour dans le tractus gastro-intestinal ne sont pas non plus satisfaisants. En effet, du fait de la variabilité intra et inter individuelle du temps de résidence gastrique, la libération du(des) principe(s) actif(s) peut se produire, après que celui ci soit passé devant sa fenêtre d'absorption, qui est localisée, pour une majorité des principes actifs, dans les parties hautes du tractus gastro-intestinal. La bio-absorption peut ainsi être très faible, voire nulle.

Mais il a fallu attendre le système galénique multimicroparticulaire tel que divulgué dans la demande de brevet PCT WO-A-03/030878, pour obtenir des progrès très significatifs, notamment au regard des problèmes susvisés de chronothérapie et de maintien d'un profil plasmatique élevé le plus longtemps possible. Ce système à libération retardée, contrôlée et certaine du(des) principe(s) actif(s), est caractérisé par un double mécanisme de déclenchement de la libération du(des) principe(s) actif(s): libération "temps dépendant" déclenchée au bout d'une durée contrôlée dans l'estomac, sans changement de pH et libération "pH dépendant" déclenchée par une remontée du pH, lorsque la forme galénique pénètre dans l'intestin. Ces deux facteurs déclencheurs de la libération du(des) principe(s) actif(s) sont mis en série et confèrent au système galénique une grande sécurité d'emploi. La libération du(des) principe(s) actif(s) est ainsi garantie après un temps de latence préréglé, même si la variation de pH n'est pas intervenue comme déclencheur, c'est-à-dire même si la forme galénique n'est pas passée de l'estomac dans l'intestin. Ces microcapsules de diamètre compris entre 200 et 600 microns sont caractérisées par une pellicule d'enrobage à base d'un polymère hydrophile A de type EUDRAGIT®L associé à un composé B hydrophobe, tel qu'une cire végétale (LUBRITAB ®) de température de fusion comprise entre 40 et 90°C, le rapport B/A = 0,2-1,5. Ces microcapsules ont un comportement de dissolution in vitro tel qu'à pH 1,4 constant, on observe une phase de latence comprise entre 1 et 5 heures, suivie d'une phase de libération du principe actif et tel que le passage de pH 1,4 à pH 6,8 entraîne une libération du principe actif sans temps de latence in vitro.

Le système galénique multimicroparticulaire selon la demande de brevet PCT WO-A-03/030878 permet aussi d'ajuster le temps de latence précédant la libération du PA dans l'estomac en prenant en considération les conditions physiologiques du tractus gastro-intestinal chez l'homme. Cette modalité avantageuse est ainsi un moyen de minimiser la variabilité interindividuelle de l'absorption du PA. En effet, d'après les résultats bien connus de Davis et al., J. of Controlled Release, 2, 27-38 (1985), le temps de résidence dans l'estomac d'une préparation est très variable, de l'ordre de 0,5 à 10 heures. Or, précisément, le système susvisé permet de libérer le principe actif dans l'estomac après un temps de latence donné, constant et compris dans cet intervalle 0,5-10 heures de façon à ce que, d'un individu à l'autre, ou même d'un jour à l'autre pour le même individu, le temps d'action du médicament soit le même.

En définitive, la forme galénique orale microparticulaire à libération retardée et contrôlée de PA, selon le WO-A-03/03878 a simultanément les propriétés suivantes :
▪ la libération du PA peut se déclencher de deux façons :
   ∘ par libération dépendante du temps également dénommée "temps dépendant" lorsque la durée de séjour des particules dans l'estomac excède une durée de 5 heures ;
   ∘ par libération dépendante d'une variation de pH, également dénommée "pH dépendant", qui débute sans temps de latence lorsque le système pénètre dans l'intestin et que le pH s'accroît. Ces deux facteurs déclencheurs de la libération de PA mis en série garantissent la libération du PA après un temps de latence préréglé, même si la variation de pH n'est pas intervenue comme déclencheur ;
▪ elle est constituée d'une pluralité de microcapsules de PA enrobé, de petite taille ;
▪ la fraction massique en excipients d'enrobage est limitée.

Il est à noter que le problème de maintien d'un profil plasmatique élevé le plus longtemps possible, peut être résolu, conformément à l'invention selon le WO-A-03/03878, par la mise en oeuvre d'un mélange de microcapsules de profils de libération retardée et contrôlée différents. Cela permet de réaliser des profils de libération présentant plusieurs vagues de libération ou assurant par un réglage adéquat des différentes fractions, un niveau constant de concentration plasmatique du(des) principe(s) actif(s).

### Problème technique

Il n'en reste pas moins que cette forme galénique orale microparticulaire à libération retardée et contrôlée de principe(s) actif(s), selon le WO-A-03/03878, peut encore être améliorée.

En effet, on sait que pour être libéré, un principe actif microencapsulé doit tout d'abord être atteint par les liquides du tractus gastrointestinal, qui doivent pour ce faire franchir la pellicule d'enrobage des microcapsules. Le principe actif microencapsulé peut alors se dissoudre dans ces liquides. La solution de principe actif ainsi obtenue peut ensuite diffuser vers l'extérieur au travers de la (ou des) pellicule(s) d'enrobage des microcapsules. Ainsi, pour obtenir un temps de latence compris entre 0,5 et 7 h -par exemple de 2-3 h-, il importe que la pellicule d'enrobage des microcapsules ait une épaisseur suffisante (en µm) et/ou représente un taux de pelliculage TP (en % en poids) suffisant afin que le temps d'entrée d'un liquide tel que l'eau ou un liquide du tractus gastro-intestinal dans la microcapsule permette la libération retardée du principe actif. Cette épaisseur plancher peut, par exemple, correspondre à un TP de 10-40 %, par exemple.

Cependant, lorsque l'épaisseur du pelliculage est suffisante pour conduire à un temps de latence, il a pu être constaté que, dans le cas des principes actifs peu solubles, la libération "temps dépendante", tout comme la libération "pH dépendante" du principe actif, sont toujours aussi efficaces, mais deviennent plus lentes, ce qui peut nuire à la biodisponibilité. Par exemple, au moins 80% en poids du principe actif, ne sont pas libérés après par exemple 16 h à pH= 7,0, dans un test de dissolution in vitro réalisé selon les indications de la pharmacopée européenne 4ème édition intitulée : "Essai de la dissolution des formes orales solides" : dissolutest de type II effectué en conditions SINK maintenu à 37°C et agité à 100 tours/min.

Il existe donc à ce jour un besoin pour une composition pharmaceutique ou un médicament oral multimicrocapsulaire, à libération modifiée de principe(s) actif(s), qui soit du type de ceux divulgués dans WO-A-03/030878 et qui perfectionne ceux-ci, en permettant notamment d'obtenir, pour les principes actifs peu solubles, une libération du principe actif selon un double mécanisme de déclenchement "temps dépendant" et "pH dépendant", avec des temps de libération plus rapides qui permettent d'optimiser la bioabsorption du(ou des) principe(s) actif(s), quel que soit le mécanisme de déclenchement de cette libération.

### Objectifs

L'un des objectifs essentiels de l'invention est de fournir un médicament oral perfectionné par rapport à celui décrit dans le WO-A-03/03878, précisément un médicament oral, multimicrocapsulaire et à libération modifiée de principe(s) actif(s) -en particulier de principe(s) actif(s) peu soluble(s)-, qui garantisse un bon fonctionnement du double mécanisme de déclenchement "temps dépendant" et "pH dépendant" de la libération du principe actif, en particulier pour des principes actifs peu solubles.

Un autre objectif essentiel de l'invention est de fournir un médicament oral multimicrocapsulaire à libération modifiée de principe(s) actif(s), permettant d'ajuster judicieusement la cinétique de libération du principe actif tout au long de sa fenêtre d'absorption dans le tractus gastro-intestinal afin que le profil de concentration plasmatique se maintienne au dessus de la concentration thérapeutique efficace pendant le plus longtemps possible, et en particulier pendant une durée supérieure à celle de la forme à libération immédiate.

Un autre objectif essentiel de l'invention est de fournir un médicament oral multimicrocapsulaire à libération modifiée de principe(s) actif(s), qui propose une solution adaptée au problème de chronothérapie et aux difficultés d'observance y afférentes.

Un autre objectif essentiel de l'invention est de fournir un médicament oral multimicrocapsulaire à libération modifiée de principe(s) actif(s), permettant de combiner facilement au moins deux principe(s) actif(s) dans une même forme pharmaceutique.

Un autre objectif essentiel de l'invention est de fournir un médicament oral multimicrocapsulaire à libération modifiée de principe(s) actif(s), qui offre, contrairement aux formes monolithiques compactes, une variabilité inter-individuelle réduite.

Un autre objectif essentiel de l'invention est de fournir un médicament oral multimicrocapsulaire à libération modifiée de principe(s) actif(s) qui permette un accroissement du temps de transit gastro-intestinal et une absorption du principe actif dans les parties hautes du tractus gastro-intestinal.

Un autre objectif essentiel de l'invention est de fournir un médicament oral multimicrocapsulaire à libération modifiée de principe(s) actif(s), ce médicament pouvant exister sous une présentation administrable une fois par jour, représenterait un progrès significatif, notamment en termes d'observance pour les patients.

Un autre objectif essentiel de l'invention est de fournir un médicament oral, multimicrocapsulaire et à libération modifiée de principe(s) actif(s) qui puisse être produit selon un procédé industriel robuste.

Un autre objectif essentiel de l'invention est de fournir un médicament oral multimicrocapsulaire , à libération modifiée de principe(s) actif(s) , qui soit aisé à préparer, par exemple par dépôt d'un revêtement par pulvérisation sur des microparticules contenant du principe actif peu soluble.

Un autre objectif essentiel de l'invention est de fournir un médicament oral multimicrocapsulaire, à libération modifiée de principe(s) actif(s) et susceptibles de présenter des teneurs élevées en principe(s) actif(s), par exemple jusqu'à 60% en poids des microcapsules.

Un autre objectif essentiel de l'invention est de fournir un médicament oral à libération modifiée de principe(s) actif(s), contenant une pluralité de microcapsules et ayant un profil de libération in vitro du principe actif indépendant de la dose.

Un autre objectif essentiel de l'invention est de fournir un médicament oral multimicrocapsulaire, à libération modifiée de principe(s) actif(s), dans lequel les microcapsules sont non entériques, c'est-à-dire ne libérant pas le principe actif seulement lorsque le pH passe de 1,4 à 7,0 (pH gastrique ⇒ pH intestinal).

Un autre objectif essentiel de l'invention est de fournir un médicament oral multimicrocapsulaire, à libération modifiée de principe(s) actif(s), permettant d'obtenir une concentration plasmatique (C24h) en principe(s) actif(s) 24 h après l'administration per os, la plus élevée possible.

Un autre objectif essentiel de l'invention est de fournir des microcapsules à libération modifiée de principes actifs, utilisables notamment pour préparer un médicament tel que défini par les spécifications énoncées dans les objectifs ci-dessus.

### Bref exposé de l'invention

Ces objectifs, parmi d'autres, sont atteints par l'invention qui concerne, en premier lieu, un médicament oral comprenant une pluralité de microcapsules à libération modifiée de principe(s) actif(s), au moins une partie desdites microcapsules étant individuellement constituée d'une microparticule comprenant au moins un principe actif- en particulier au moins un principe actif peu soluble- (à l'exclusion du carvédilol) enrobée d'au moins un revêtement permettant la libération modifiée du(des) principe(s) actif(s), ladite libération étant régie par deux mécanismes distincts de déclenchement, l'un étant basé sur une variation de pH et l'autre permettant la libération du(des) principe(s) actif(s), au bout d'un temps prédéterminé de résidence dans l'estomac,

ledit revêtement conférant également aux microcapsules un comportement de dissolution in vitro tel que :
- à pH constant 1,4, le profil de dissolution comporte une phase de latence de durée inférieure ou égale à 7 heures, de préférence inférieure ou égale à 5 heures, et, plus préférentiellement encore comprise entre 1 à 5 heures;
- le passage de pH 1,4 à pH 7,0, conduit à une phase de libération débutant sans temps de latence;
   ce médicament étant caractérisé
   en ce qu'au moins une partie desdites microcapsules comprend au moins un agent gonflant,
   et en ce que la fraction en poids du(ou des) principe(s) actif(s) libéré(s) pendant la phase de latence est inférieure ou égale à 15 % en poids par heure, de préférence inférieure ou égale à 10 % en poids par heure, et, plus préférentiellement encore inférieure ou égale à 5 % en poids par heure.

Le comportement de dissolution in vitro est réalisé selon les indications de la pharmacopée européenne, 4ème édition intitulée : "Essai de la dissolution des formes orales solides" : dissolutest de test de type II effectué en condition SINK, maintenu à 37° C et agité à 100 tours/min.

Le médicament selon l'invention pallie le problème technique susmentionné, à savoir la libération de principe(s) actif(s) peu soluble(s) PA selon un double mécanisme de déclenchement "temps dépendant" et "pH dépendant", en accélérant la libération du principe actif, notamment par rapport aux temps de libération obtenus par l'invention selon le WO-A-03/03878. Ce faisant, le médicament selon l'invention améliore in fine l'efficacité prophylactique et thérapeutique de tels principes actifs peu solubles.

Mais le médicament selon l'invention est également intéressant en ce qu'il offre notamment les avantages suivants:
- possibilité d'utilisation combinée simple d'au moins deux principe(s) actif(s).
- variabilité inter-individuelle réduite
- accroissement du temps de transit gastro-intestinal et absorption du principe actif dans les parties hautes du tractus gastro-intestinal
- proportionnalité entre la dose et le profil pharmacocinétique.
- facilité d'ingestion par le patient et possibilité d'administration, par exemple une seule fois par jour, ce qui est un gage de respect de l'observance et donc de garantie d'efficacité.
- reproductibilité de la cinétique de libération, d'un lot industriel à l'autre; donc développement industriel possible, sans que cela nuise aux performances thérapeutiques du(des) principe(s) actif(s) encapsulé(s) (par exemple des principes actifs peu solubles).
- préparation aisée et économique, par exemple par dépôt d'un revêtement par pulvérisation sur des microparticules contenant du principe actif peu soluble.
- possibilité d'avoir des teneurs élevées en principe(s) actif(s), par exemple jusqu'à 60% en poids des microcapsules.
- pluralité de microcapsules et ayant un profil de libération in vitro du principe actif indépendant de la dose.
- microcapsules non entériques, c'est-à-dire ne libérant pas le principe actif seulement lorsque le pH passe de 1,4 à 7,0 (pH gastrique ⇒ pH intestinal).
- concentration plasmatique en principe(s) actif(s) 24 h après l'administration per os, proche de ou supérieure à celle qui serait obtenue avec une forme à libération immédiate en plusieurs prises.

### Exposé détaillé de l'invention

Conformément à l'invention, l'agent gonflant comprend au moins un composé, pharmaceutiquement acceptable, hydrophile et présentant un taux de gonflement dans l'eau à 25°C, supérieur ou égal à 10 % en poids, de préférence supérieur ou égal à 15 % en poids et plus préférentiellement encore supérieur ou égal à 20%.

Suivant une caractéristique remarquable de l'invention, l'agent gonflant est choisi parmi ceux permettant aux microcapsules de libérer au moins 50% en poids du principe actif, après 16 h à pH= 1,4 et après une phase de latence de durée inférieure ou égale à 7 heures, de préférence inférieure ou égale à 5 heures,et, plus préférentiellement encore comprise entre 1 à 5 heures, dans un test de dissolution in vitro réalisé selon les indications de la pharmacopée européenne 4ème édition intitulée : "Essai de la dissolution des formes orales solides" : dissolutest de type II effectué en conditions SINK maintenu à 37 C et agité à 100 tours/min.

Conformément à l'invention, il est possible d'ajuster la vitesse de libération à pH= 1,4 du (ou des) principe(s) actif(s) hors des microcapsules en choisissant judicieusement la concentration (Cd) d'agent gonflant.

Lorsque l'agent gonflant est sous forme microparticulaire, le choix de la taille (Td) des particules d'agent gonflant se fait avantageusement dans les intervalles de diamètre moyen en µm, compris entre 5 et 200 µm et, de préférence, entre 10 et 50 µm.

Le choix de la concentration (Cd) en agent gonflant se fait dans les intervalles suivants de % en poids par rapport à la masse totale des microcapsules:
- 3 ≤ Cd ≤ 40,
- de préférence 4 ≤ Cd ≤ 30,
- et plus préférentiellement encore 5 ≤ Cd ≤ 25.

Selon un mode préféré de réalisation de l'invention, l'agent gonflant est choisi dans le groupe de produits suivants :
∘ les polyvinylpyrrolidones réticulées (e.g. polyplasdone ou crospovidone),
∘ les carboxyalkylcelluloses réticulées: les carboxyméthylcelluloses réticulées (e.g. croscarmellose de sodium réticulée),
∘ ainsi que les polymères hydrophiles de haute masse molaire (supérieure ou égale à 100.000 D) tels que:
   ▪ les polyvinylpyrrolidones,
   ▪ les polyalkylènes oxydes (e.g. polyéthylène oxyde ou de polypropylène oxyde,
   ▪ les (hydroxy)(alkyl)celluloses (e.g. hydroxypropylcellulose, hydroxypropylméthylcellulose),
   ▪ les carboxyalkylcelluloses (e.g. carboxyméthylcellulose),
   ▪ les celluloses (poudre ou microcristalline),
   ▪ les amidons modifiés (par exemple avec du glycolate de sodium),
   ▪ les amidons natifs (par exemple de maïs, de blé ou de pomme de terre),
   ▪ l'alginate de sodium,
   ▪ la polacriline de potassium,
   ▪ et leurs mélanges.

De manière plus préférée encore, l'agent gonflant est choisi dans le sous-groupe de produits suivants :
o les polyvinylpyrrolidones réticulées (e.g. polyplasdone ou crospovidone), et
o les carboxyalkylcelluloses réticulées: les carboxyméthylcelluloses réticulées (e.g. croscarmellose de sodium réticulée).

Pour pallier l'éventualité où des principes actifs (par exemple peu solubles) seraient mal mouillés par l'eau et auraient donc tendance à s'agglomérer, il est proposé suivant une variante avantageuse de l'invention, de faire en sorte que le médicament comprenne au moins un agent mouillant, de préférence choisi dans le groupe de produits suivants :
∘ les tensioactifs anioniques, de préférence dans le sous-groupe des sels alcalins ou alcalinoterreux des acides gras, l'acide stéarique et/ou oléique étant préférés,
∘ et/ou les tensioactifs non ioniques, de préférence dans le sous-groupe suivant:
   ▪ les huiles polyoxyéthylénées de préférence l'huile de ricin hydrogénée polyoxyéthylénée,
   ▪ les copolymères polyoxyéthylène-polyoxypropylène,
   ▪ les esters de sorbitan polyoxyéthylénés,
   ▪ les dérivés de l'huile de ricin polyoxyéthylénés,
   ▪ les stéarates, de préférence de calcium, de magnésium, d'aluminium ou de zinc,
   ▪ les stéarylfumarates, de préférence de sodium,
   ▪ le béhénate de glycérol,
   ▪ et leurs mélanges.

Avantageusement, le médicament selon l'invention comprend des microcapsules de principe(s) actif(s), aptes à libérer au moins 80% en poids du (ou des) principe(s) actif(s), après 12 h à pH= 7,0, dans un test de dissolution in vitro réalisé selon les indications de la pharmacopée européenne 4ème édition intitulée : "Essai de la dissolution des formes orales solides" : dissolutest de type II effectué en conditions SINK maintenu à 37 C et agité à 100 tours/min.

Le médicament selon l'invention est multimicrocapsulaire, c'est-à-dire qu'il comprend entre autres des microcapsules constituées de microparticules de principe actif enrobé ou pelliculé. Ces microparticules de principe actif peuvent être, par exemple, du principe actif brut (pur) sous forme pulvérulente, des granules matriciels de principe actif avec différents autres ingrédients ou bien encore des microsphères neutres, par exemple en cellulose ou en sucre, recouvertes d'au moins une couche comportant du principe actif.

Les microcapsules de principe actif à libération modifiée sont assimilables à des micro-unités contenant au moins un principe actif et formant au moins une partie des éléments constitutifs du médicament selon l'invention.

Chaque microcapsule peut comprendre un ou plusieurs principes actifs identiques ou différents entre eux.

Le médicament selon l'invention peut comprendre des micro-unités de principe actif autres que des microcapsules. Il pourrait s'agir, par exemple, de microparticules à libération immédiate de principe(s) actif(s). Ces dernières peuvent être e.g. des microparticules de principe(s) actif(s) non enrobées du même type que celles utiles dans la préparation des microcapsules selon l'invention.

Chaque microparticule peut comprendre un ou plusieurs principes actifs identiques ou différents entre eux.

En outre, l'ensemble des micro-unités (microparticules et/ou microcapsules) constituant le médicament selon l'invention peut être formé par différentes populations de micro-unités, ces populations différant entre elles au moins par la nature du(ou des) principe(s) actif(s) contenus dans ces micro-unités et/ou par la composition du revêtement.

S'agissant de la structure des microcapsules mises en oeuvre dans le médicament selon l'invention, on détaille ci-après, à titre non limitatif, deux formes préférées de réalisation de la structure des microcapsules.

Selon une première forme de réalisation, au moins une partie des microcapsules à libération modifiée de principe(s) actif(s) comporte chacune:
o une microparticule de principe(s) actif(s), enrobée par
o au moins un revêtement permettant la libération modifiée du (ou des) principe(s) actif(s).

De préférence, la microparticule de principe(s) actif(s) est un granulé comprenant le(les) principe(s) actif(s) et un ou plusieurs excipients pharmaceutiquement acceptables.

Avantageusement, le(ou les) agent(s) gonflant(s) est (sont) contenu(s) dans la microparticule (e.g. granulé).

Quant à l'(ou les) agent(s) mouillant(s), il(s) est (sont) préférablement contenu(s) dans la microparticule (e.g. granulé) et/ou dans le revêtement permettant la libération modifiée du (ou des) principe(s) actif(s).

Selon une deuxième forme de réalisation, au moins une partie des microcapsules à libération modifiée de principe(s) actif(s) comporte chacune:
o un coeur neutre,
o au moins une couche active comprenant le (ou les) principe(s) actif(s) et enrobant le coeur neutre, et
o au moins un revêtement permettant la libération modifiée du
o (ou des) principe(s) actif(s).

Suivant une première possibilité, le coeur neutre contient du sucrose et/ou du dextrose et/ou du lactose.

Suivant une deuxième possibilité, le coeur neutre est une microsphère de cellulose.

Avantageusement, le coeur neutre a un diamètre moyen compris entre 1 et 800 µm et de préférence compris entre 20 et 500 µm.

La couche active peut éventuellement comporter, outre le (ou les) principe(s) actif(s), un ou plusieurs excipients pharmaceutiquement acceptables.

Avantageusement, l'(ou les) agent(s) gonflant(s) est (sont) contenu(s) dans la couche active.

Par exemple, cette couche active comprend du principe actif, au moins un agent gonflant, au moins un liant et au moins un tensioactif.

Quant à l'(ou les) agent(s) mouillant(s), il(s) est (sont) préférablement contenu(s) dans la couche active.

Concernant maintenant la composition du revêtement des microcapsules à libération modifiée de principe(s) actif(s), la présente invention a consisté également à sélectionner des microcapsules ayant les spécificités suivantes:
∘ le revêtement permettant la libération modifiée du (ou des) principe(s) actif(s) comprend un matériau composite
   ▪ comportant :
      - au moins un polymère hydrophile A porteur de groupements ionisés à pH neutre,
      - au moins un composé hydrophobe B ;
   ▪ représentant une fraction massique (% poids par rapport à la masse totale des microcapsules) ≤ 40 ; et
∘ leur diamètre moyen inférieur à 2000 µm, et de préférence compris entre 50 et 800 µm et, plus préférentiellement encore, entre 100 et 600 µm.

Selon une autre caractéristique avantageuse, le matériau composite AB du revêtement permettant la libération modifiée du principe actif peu soluble,est tel que:
∘ le ratio pondéral B/A, est compris entre 0,2 et 1,5, de préférence entre 0,5 et 1,0,
∘ et le composé B hydrophobe est sélectionné parmi les produits cristallisés à l'état solide et ayant une température de fusion TfB ≥ 40°C, de préférence TfB ≥ 50°C, et plus préférentiellement encore 40°C ≤ TfB ≤ 90°C.

Selon un mode de mise en oeuvre de prédilection, le polymère hydrophile A est choisi parmi :
- A.a les copolymères d'acide (méth)acrylique et d'ester alkyle d'acide (méth)acrylique et leurs mélanges;
- A.b les dérivés de la cellulose, de préférence les acétates de celluloses, les phtalates de celluloses, les succinates de celluloses et leurs mélanges, et, plus préférentiellement encore les phtalates d'hydroxypropylméthyl-celluloses, les acétates d'hydroxypropyl-méthylcelluloses, les succinates d'hydroxypropylméthylcelluloses et leurs mélanges;
- et leurs mélanges.

Les polymères A encore plus préférés sont des copolymères d'acide(méth)-acryliques et d'esters d'alkyle (e.g. alkyle en C1-C6) d'acide(méth)acrylique. Ces copolymères sont, par exemple, du type de ceux commercialisés par la société Röhm Pharma Polymers sous les marques déposées EUDRAGIT®, des séries L et S (comme par exemple les EUDRAGIT® L100, S100, L30 D-55 et L100-55). Ces copolymères sont des copolymères entériques anioniques et solubles en milieu aqueux à des pH supérieurs à ceux rencontrés dans l'estomac.

Toujours selon le mode de mise en oeuvre de prédilection, le composé B est choisi parmi le groupe de produits suivants :
- B.a cires végétales prises à elles seules ou en mélanges entre-elles;
- B.b huiles végétales hydrogénées prises à elles seules ou en mélange entre-elles;
- B.c mono et/ou di et/ou tri esters du glycérol et d'au moins un acide gras;
- B.d mélanges de monoesters, de diesters et de triesters du glycérol et d'au moins un acide gras;
- B.e et leurs mélanges.

De manière plus préférée encore, le composé B est choisi parmi le groupe de produits suivants : huile hydrogénée de graines de coton, huile hydrogénée de graines de soja, huile de palme hydrogénée, béhénate de glycérol, huile de ricin hydrogénée, tristéarine, tripalmitine, trimyristine, cire jaune, matière grasse dure ou matière grasse utile comme bases de suppositoires, matières grasses laitières anhydres, lanoline, palmitostéarate de glycerol, stéarate de glycérol, macrogolglycérides lauryliques, alcool cétylique, diisostéarate de polyglycérol, diéthylène glycol monostéarate, éthylène glycol monostéarate, Oméga 3 et tout mélange d'entre eux,
de préférence dans le sous-groupe de produits suivants: huile hydrogénée de graines de coton, huile hydrogénée de graines de soja, huile de palme hydrogénée, béhénate de glycérol, huile de ricin hydrogénée, tristéarine, tripalmitine, trimyristine et tout mélange d'entre eux.

En pratique et sans que cela ne soit limitatif, on préfère que le composé B soit choisi:
∘ dans le groupe de produits commercialisés sous les marques suivantes : Dynasan®, Cutina®, Hydrobase®, Dub®, Castorwax®, Croduret®, Compritol®, Sterotex®, Lubritab®, Apifil®, Akofine®, Softtisan®, Hydrocote®, Livopol®, Super Hartolan®, MGLA®, Corona®, Protalan®, Akosoft®, Akosol®, Cremao®, Massupol®, Novata®, Suppocire®, Wecobee®, Witepsol®, Lanolin®, Incromega®, Estaram®, Suppoweiss®, Gelucire®, Precirol®, Emulcire®, Plurol diisostéarique®, Geleol®, Hydrine® et Monthyle® et leurs mélanges;
∘ ainsi que dans le groupe d'additifs dont les codes sont les suivants : E 901, E 907, E 903 et leurs mélanges;
∘ et, de préférence dans le groupe de produits commercialisés sous les marques suivantes: Dynasan® P60, Dynasan® 114, Dynasan® 116, Dynasan® 118, Cutina® HR, Hydrobase® 66-68, Dub® HPH, Compritol® 888, Sterotex® NF, Sterotex® K, Lubritab® et leurs mélanges.

Selon une autre caractéristique intéressante de l'invention, le revêtement permettant la libération modifiée du principe actif peu soluble est exempt de talc.

Suivant une autre caractéristique remarquable relevant de la préparation des microcapsules, le principe actif est déposé sur le coeur par les techniques connues de l'homme de l'art, par exemple la technique de "spray coating" en lit d'air fluidisé, la granulation humide, le compactage, l'extrusion-sphéronisation ....

Avantageusement, l'enrobage des microcapsules peut comprendre, outre les constituants essentiels A et B, d'autres ingrédients classiques et connus de l'homme du métier, tels que notamment :
∘ des colorants,
∘ des plastifiants comme par exemple le dibutylsébaçate,
∘ des composés hydrophiles, comme par exemple la cellulose et ses dérivés ou la polyvinylpirrolidone et ses dérivés,
∘ et leurs mélanges.

Sans que cela ne soit limitatif et selon une forme de réalisation encore plus préférée, le revêtement des microcapsules à libération modifiée de principe actif comprend une seule pellicule d'enrobage composite AB.

Sur le plan quantitatif, la monocouche d'enrobant peut représenter, par exemple, au plus 40 %, de préférence au plus 30 % en poids des microcapsules. Un tel taux limité d'enrobage permet de réaliser des unités galéniques contenant chacune une haute dose de principe actif, sans dépasser une taille rédhibitoire au regard de la déglutition. L'observance et donc le succès du traitement ne peuvent que s'en trouver améliorés.

Le mécanisme de déclenchement de la libération du principe actif peu soluble sans variation de pH, au bout d'un temps prédéterminé de résidence dans l'estomac, résulte notamment du contrôle de la vitesse d'hydratation des microcapsules et/ou de la dissolution d'un ou plusieurs composants des microcapsules. Par exemple, et sans vouloir être limitatif, l'hydratation de la microcapsule peut être contrôlée :
∘ par la présence, dans les microcapsules, de produits hydrophiles qui permettent d'ajuster la pression osmotique ou de provoquer un gonflement des microcapsules,
∘ ou par le réglage de la perméabilité à l'eau de la pellicule d'enrobage;
∘ ou par la création d'une microporosité dans la pellicule d'enrobage,
∘ ou même par l'hydratation ou la dissolution d'un composé de la pellicule d'enrobage.

L'un des avantages déterminants du système galénique multimicrocapsulaire, à libération retardée et contrôlée de principe(s) actif(s) -par exemple de principe(s) peu soluble(s)- selon l'invention, est de faire intervenir in vivo deux facteurs déclencheurs de la libération du (des) principe(s) actif(s) -par exemple du (des) principe(s) peu soluble(s)-dans le tractus gastro-intestinal, à savoir :
- la durée de séjour dans l'estomac : libération "temps déclenchée",
- la variation de pH : libération "pH déclenchée".

Ces deux facteurs déclencheurs de la libération de principe(s) actif(s) -par exemple de principe(s) peu soluble(s)- sont en série, de sorte qu'ils confèrent au système galénique une grande sécurité d'emploi. La libération du (des) principe(s) actif(s) -par exemple du (des) principe(s) peu soluble(s)-est ainsi garantie après un temps de latence préréglé, même si la variation de pH n'est pas intervenue comme déclencheur. Les problèmes de variabilité interindividuelle sont ainsi surmontés. L'efficacité thérapeutique du médicament comprenant un tel système galénique est assurée, en respectant une chronobiologie prédéterminée et adaptée à la performance thérapeutique visée.

En outre, pour tout principe actif (e.g. peu soluble) dont la fenêtre d'absorption est limitée aux parties hautes du tractus gastro-intestinal, il est particulièrement avantageux que la forme à libération retardée puis prolongée et contrôlée soit formée par une pluralité de microcapsules. En effet, pour une telle forme galénique, la dose de principe actif (e.g. peu soluble) à administrer se répartit entre un grand nombre de microcapsules (typiquement 5000 - 50000) et présente de ce fait les avantages intrinsèques suivants :
∘ Le temps de séjour des microcapsules dans les parties hautes du tractus gastro-intestinal peut être prolongé, ce qui assure un accroissement de la durée de passage du principe actif peu soluble devant les fenêtres d'absorption et maximise ainsi la biodisponibilité du principe actif peu soluble.
∘ La mise en oeuvre d'un mélange de microcapsules de profils de libération retardée et contrôlée différents, permet de réaliser des profils de libération présentant plusieurs vagues de libération ou assurant par un réglage adéquat des différentes fractions, un niveau de concentration plasmatique du principe actif peu soluble constant.
∘ La variabilité de la vidange gastrique est moindre, car la vidange qui s'effectue ici sur un grand nombre de particules est statistiquement plus reproductible.
∘ On évite la mise en contact des tissus avec une dose élevée en principe actif peu soluble : "dose dumping ". Chaque microcapsule ne contient en effet qu'une dose très réduite en principe actif peu soluble. On s'affranchit ainsi du risque de détérioration des tissus par surconcentration locale de principe actif peu soluble agressif
∘ Il est possible de présenter ces microcapsules sous forme de sachet, gélule ou comprimé. Dans les cas où la dose de principe actif peu soluble est élevée (500 mg ou plus) les formes monolithiques sont de trop grandes dimensions pour être facilement avalées. Il est alors particulièrement intéressant de disposer d'une forme microparticulaire qui assure la libération retardée et contrôlée du principe actif (en particulier peu soluble) et que l'homme de l'art peut mettre en forme de comprimés délitables ou de sachets.

Le système galénique multimicrocapsulaire selon l'invention permet d'assurer de manière sûre une libération retardée et contrôlée du principe actif peu soluble dans le tractus gastro-intestinal, grâce à deux déclencheurs et de se soustraire ainsi à la variabilité inter et intra individuelle des conditions de la vidange gastrique, tout en étant viable économiquement et facile à ingérer (observance optimisée).

Un autre objet de l'invention concerne un médicament oral comprenant une pluralité de microcapsules à libération modifiée de principe(s) actif(s), au moins une partie desdites microcapsules étant individuellement constituée d'une microparticule comprenant au moins un principe actif -en particulier au moins un principe actif peu soluble-(à l'exclusion du carvédilol) enrobée d'au moins un revêtement permettant la libération modifiée du(des) principe(s) actif(s), ladite libération étant régie par deux mécanismes distincts de déclenchement, l'un basé sur une variation de pH et l'autre permettant la libération du(ou des) principe(s) actif(s), au bout d'un temps prédéterminé de résidence dans l'estomac, ledit revêtement:
▪ conférant également aux microcapsules un comportement de dissolution in vitro (réalisé selon les indications de la pharmacopée européenne 4^{ème} édition intitulée : "Essai de la dissolution des formes orales solides" : dissolutest de type II effectué en conditions SINK maintenu à 37 C et agité à 100 tours/min), tel que :
   - à pH constant 1,4, le profil de dissolution comporte une phase de latence de durée inférieure ou égale à 7 heures, de préférence inférieure ou égale à 5 heures,et, plus préférentiellement encore comprise entre 1 à 5 heures ;
   - au moins 50% en poids du(ou des) principe(s) actif(s) sont libérés après 16h à pH 1,4;
   - le passage de pH 1,4 à pH 7,0, conduit à une phase de libération débutant sans temps de latence;
▪ et comprenant un matériau composite comportant au moins un polymère hydrophile A porteur de groupements ionisés à pH neutre et au moins un composé hydrophobe B ; ce médicament étant caractérisé
▪ en ce qu'au moins une partie desdites microcapsules comprend au moins un auxiliaire de libération apte à augmenter la perméabilité du revêtement permettant la libération modifiée du(ou des) principe(s) actif(s),
▪ et en ce que la fraction en poids du(ou des) principe(s) actif(s) libéré(s) pendant la phase de latence est inférieure ou égale à 15 % en poids par heure, de préférence inférieure ou égale à 10 % en poids par heure, et, plus préférentiellement encore inférieure ou égale à 5 % en poids par heure.

Il est en effet apparu utile conformément à l'invention de prévoir dans les microcapsules, un ou plusieurs auxiliaires propres à augmenter la perméabilité du revêtement, de façon à réduire le temps de libération notamment pour les principes actifs peu solubles.

Avantageusement, l'auxiliaire de libération est constitué par au moins un agent gonflant, tel que défini supra.

Le médicament selon cet autre objet de l'invention, a également ceci de singulier que le revêtement des microcapsules comprises dans ce médicament, confère auxdites microcapsules un comportement de dissolution in vitro (réalisé selon les indications de la pharmacopée européenne 4ème édition intitulée : "Essai de la dissolution des formes orales solides" : dissolutest de type II effectué en conditions SINK maintenu à 37°C et agité à 100 tours/min),
tel qu'au moins 50% en poids du(ou des) principe(s) actif(s) sont libérés après 16h à pH 1,4.

Le fait que le médicament selon l'invention soit constitué d'une pluralité de micro-unités, permet d'accéder à une autre caractéristique essentielle de l'invention selon laquelle le médicament comprend un mélange de différentes populations de micro-unités contenant du(ou des) principe(s) actif(s) (à l'exclusion du carvédilol), ces populations différant entre elles par leurs profils de dissolution in vitro respectifs, pour au moins une valeur de pH comprise entre 1,4 et 7,4.

Cette caractéristique essentielle permet d'obtenir une augmentation du temps de bioabsorption du(des) principe(s) actif(s) et donc du temps pendant lequel la concentration plasmatique est supérieure à la concentration minimale efficace de ce principe actif.

En fait, le mélange de différentes populations de micro-unités (e.g. microcapsules à libération modifiée de principe actif et éventuellement microparticules à libération immédiate de principe actif) conduit à une libération préférentielle du(ou des) principe(s) actif(s) en des endroits différents du tractus gastro intestinal, sur toute l'étendue de la fenêtre de bio-absorption du(ou des) principe(s) actif(s) dans le tractus gastro intestinal.

Selon une forme avantageuse de mise en pratique de cette caractéristique de mélange de diverses populations de micro-unités, le médicament selon l'invention est caractérisé en ce que les micro-unités sont des microcapsules à libération modifiée de principe(s) actif(s) et éventuellement des micro-unités à libération immédiate de principe(s) actif(s).

Avantageusement, les populations de microcapsules à libération modifiée de(s) principe(s) actif(s) diffèrent par leurs pH de déclenchement respectifs.

Les populations de microcapsules à libération modifiée de(s) principe(s) actif(s) peuvent non seulement différer par leurs pH de déclenchement respectifs mais également par leurs temps de déclenchement respectifs, voire par leurs pH et leurs temps de déclenchement respectifs.

Selon une forme préférée de mise en pratique de cette caractéristique de mélange de populations, le médicament comprend :
i. au moins une population de micro-unités contenant du principe actif à libération immédiate;
ii. au moins une population P1 de microcapsules à libération modifiée de(s) principe(s) actif(s); et
iii. au moins une population P2 de microcapsules à libération modifiée de(s) principe(s) actif(s);
et, par ailleurs, les pH de déclenchement respectifs de P1 et de P2 diffèrent d'au moins 0,5 unités de pH, de préférence d'au moins 0,8 unités de pH, et, plus préférentiellement encore, d'au moins 0,9 unités de pH.

Selon une disposition préférée relative aux pH de déclenchement différenciant les différentes populations de microcapsules à libération modifiée de(s) principe(s) actif(s) , lesdits pH de déclenchement respectifs sont compris entre 5 et 7.

Suivant une autre variante de la forme préférée de mise en pratique de cette caractéristique de mélange de populations, le médicament comprend :
i. au moins une population de micro-unités contenant du (des) principe(s) actif(s) à libération immédiate;
ii. au moins une population P1' de micro-unités contenant du (des) principe(s) actif(s) formées par des microcapsules à libération modifiée du (des) principe(s) actif(s), dont le pH de déclenchement est égal à 5,5; et
iii. au moins une population P2' de micro-unités contenant du (des) principe(s) actif(s) formées par des microcapsules à libération modifiée du (des) principe(s) actif(s), dont le pH de déclenchement est égal à 6,0 ou 6,5.

Le profil de libération (mesuré dans un test de libération in vitro tel que défini ci-avant) des microcapsules à libération modifiée mises en oeuvre dans les mélanges susvisés de différentes populations de micro-unités (par exemple P1 & P2 ou P1' & P2') , peut être, par exemple, tel qu'indiqué ci-après :
▪ moins de 20 % du (des) principe(s) actif(s) sont libérés au bout de 2 heures à pH = 1,4;
▪ au moins 50 % du (des) principe(s) actif(s) sont libérés au bout de 16 heures à pH = 1,4.

Dans le cas où le médicament selon l'invention comprend au moins une population de micro-unités contenant du (des) principe(s) actif(s) à libération immédiate, cette population peut avantageusement être définie par son comportement dans un test de dissolution in vitro, ledit comportement étant tel qu'au moins 80 % du (des) principe(s) actif(s) sont libérés en 1 heure à pH compris entre 1,4 et 7,4.

Suivant une caractéristique avantageuse de l'invention, la proportion de principe(s) actif(s) peu soluble dans les micro-unités contenant du principe(s) actif(s) (exprimée en % en poids sur sec par rapport à la masse totale des micro-unités) est comprise entre 5 et 80, de préférence entre 10 et 70, et, plus préférentiellement encore entre 15 et 60.

De préférence, les micro-unités contenant du (des) principe(s) actif(s) à libération immédiate sont des microparticules non enrobées.

Sans vouloir être limitatif, il doit être néanmoins souligné que le médicament selon l'invention est particulièrement intéressant en ce qu'il peut se présenter sous forme de dose unique orale journalière, comprenant
▪ de 5000 à 500 000 micro-unités contenant du(ou des) principe(s) actif(s), ou
▪ de 5000 à 500 000 microcapsules à libération modifiée de principe(s) actif(s).

Cette pluralité de microcapsules illustrée par les exemples numériques mentionnés ci-dessus constitue une forme galénique parfaitement bien tolérée par l'organisme de mammifères.

Ces microcapsules sont d'autant plus intéressantes que leur fabrication s'effectue de manière simple et économique selon des techniques bien connues de l'homme de l'art, par exemple la technique de spray coating en lit d'air fluidisé, la granulation humide, le compactage, l'extrusion-sphéronisation ....

Pour plus de détails sur la préparation de ces microcapsules, notamment dans leur forme de réalisation avec un coeur neutre enrobé d'au moins une couche active comprenant du (des) principe(s) actif(s) et d'au moins un revêtement extérieur permettant la libération modifiée du (des) principe(s) actif(s), on se référera au contenu de la demande PCT WO-A-FR03/030878 dont le contenu est intégré dans le présent exposé par référence.

Le médicament aux formes galéniques orales multimicroparticulaires selon l'invention peut être un comprimé, avantageusement orodispersible, une poudre, une suspension liquide ou une gélule contenant des microcapsules.

En d'autres termes, ce médicament peut se présenter sous forme de sachet de poudre de microcapsules, de suspension liquide de microcapsules, de comprimé obtenu à partir de microcapsules ou de gélule contenant des microcapsules.

Ces comprimés, ces gélules, ces poudres, ces suspensions peuvent être constitués de mélanges des différentes populations de micro-unités et notamment de microcapsules de principe(s) actif(s) selon l'invention, en y associant de préférence des micro-unités ou micro particules à libération immédiate de principe actif peu soluble (par exemple des granules).

Par ailleurs, l'invention vise l'utilisation des microcapsules à libération modifiée du (des) principe(s) actif(s) tel que défini ci-dessus et éventuellement des micro-unités contenant du (des) principe(s) actif(s) à libération immédiate pour la préparation de formes galéniques orales microparticulaires, pharmaceutiques ou diététiques, de préférence sous forme de comprimés avantageusement orodispersibles, de poudres ou de gélules.

De plus, l'invention concerne les microcapsules telles que définies ci-dessus prises en tant que telles.

De préférence, le (ou les) principe(s) actif(s) peu(ven)t être choisi(s) parmi au moins l'une des grandes variétés de substances actives, suivantes e.g. : antiulcéreux, antidiabétiques, anticoagulants, antithrombiques, hypolipémiants, antiarythmiques, vasodilatateurs, antiangineux, antihypertenseurs, vasoprotecteurs, promoteurs de fécondité, inducteurs et inhibiteurs du travail utérin, contraceptifs, antibiotiques, antifongiques, antiviraux, anticancéreux, anti-inflammatoires, analgésiques, antiépileptiques, antiparkinsoniens, neuroleptiques, hypnotiques, anxiolytiques, psychostimulants, antimigraineux, antidépresseurs, antitussifs, antihistaminiques ou antiallergiques, agents permettant de lutter contre l'insuffisance cardiaque congestive, l'angine de poitrine, l'hypertrophie ventriculaire gauche, les arythmies cardiaques, les infarctus du myocarde, la tachycardie réflexe, la maladie cardiaque ischémique, l'athéromatose, l'hypertension associée au diabète mellitus, l'hypertension portale, les vertiges, l'abradicardie, l'hypotension artérielle, la rétention hydrosodée, l'insuffisance rénale aigue, l'hypotension orthostatique et la congestion cérébrale, et leurs mélanges.

Comme exemples de principes actifs qui peuvent être contenus dans le médicament selon l'invention, on peut citer ceux choisis dans le groupe de composés suivants : acide acétylsalycilique, carbamazepine pentoxifylline, prazosine, acyclovir, nifedipine, diltiazem, naproxen, ibuprofen, flurbiprofen, ketoprofen, fenoprofen, indométhacine, diclofenac, fentiazac, oestradiol valérate, métoprolol, sulpiride, captopril, cimetidine, zidovudine, nicardipine, terfenadine, atenolol, salbutamol, carbamazépine, ranitidine, énalapril, simvastatine, fluoxétine, alprazolam, famotidine, ganciclovir, famciclovir, spironolactone, 5-asa, quinidine, périndopril, morphine, pentazocine, paracétamol, oméprazole, lansoprazole, métoclopramide, acide aminosalicylique, acide nalidixique, amoxicilline, amoxicilline et clavulanate de potassium, ampicilline, ampicilline et sulbactame, azithromycine, bacampicilline, carbénicilline-indanyl-sodium (et autres sels de carbénicilline), capréomycine, céfadroxile, céfazoline, céphalexine, céphalothine, céphapirine, céphacelor, céphprozile, céphadrine, céfamandole, céfonicide, céforanide, céfuroxime, céfixime, céfopérazone, céfotaxime, cefpodoxime, ceftaxidime, ceftibuten, ceftizoxime, ceftriaxone, cefepime, cefmétazole, céfotetane, céfoxitine, ciprofloxacine, clarithromycoine, clindamycine, clofazimine, cloxacilline, co-triamoxazole, cyclosérine, dicloxacilline, dirithromycine, érythromycine (et sels d'érythromycine tels que estolate, éthylsuccinate, gluceptate, lactobionate, stéarate), éthambutol-HCl et autres sels, éthionamide, fosfomycine, imipenem, isoniazide, levofloxacine, lomefloxacine, loracarbef, méthicilline, méthenamine, métronidazole, métoclo-pramide, mezlocilline, nafcilline, nitrofurantoine, norfloxacine, novobiocine, ofloxacine, oxacilline, pénicilline V, sels de pénicilline, complexes de pénicilline, pentamidine, pipéracilline, pipéracilline et tazobactame, sparfloxacine, sulfacytine, sulfamérazine, sulfaméthazine, sulfaméthixole, sulfasalazine, sulfisoxazole, sulfapyrizine, sulfadiazine, sulfméthoxazole, sulfapyridine, ticarcilline, ticarcilline et clavulanate de potassium, triméthoprime, trimétrexate, troléanomycine, vancomycine, vérapamil et leurs mélanges.

Suivant une variante particulière mais non limitative de l'invention, le (ou les) principe(s) actif(s) est (sont) un (des) principe(s) actif(s) peu soluble(s), par exemple choisi(s) parmi les principes actifs tels que mentionnés ci-dessus (pris à eux seuls ou en mélange entre eux).

Les principes actifs également concernés par la présente invention, peuvent être aussi des suppléments nutritionnels et/ou diététiques ou leurs mélanges, comme par exemple des vitamines, des acides aminés, des antioxydants ou des oligoéléments ou leurs mélanges.

Enfin, l'invention vise également une méthode de traitement thérapeutique, caractérisé en ce qu'elle consiste en une administration par voie orale, selon une posologie déterminée, du médicament selon l'invention tel que défini ci-dessus.

L'invention sera mieux expliquée par les exemples ci-après, donnés uniquement à titre d'illustration afin de bien comprendre l'invention et de faire ressortir ses variantes de réalisation et/ou de mise en oeuvre.

### EXEMPLES

### Description des figures:

Figure 1: Profils de libération in vitro des microcapsules préparées selon l'exemple comparatif 1.
Figure 2: Profils de libération in vitro des microcapsules préparées selon l'exemple comparatif 2.
Figure 3: Profils de libération in vitro des microcapsules préparées selon l'exemple comparatif 3.
Figure 4: Profils de libération in vitro des microcapsules préparées selon l'exemple comparatif 4.
Figure 5 : Profils de libération in vitro des microcapsules préparées selon l'exemple 5 et comparaison avec les profils de libération des microcapsules préparées selon comparatif 1.
Figure 6: Profils de libération in vitro des microcapsules préparées selon l'exemple 6 et comparaison avec les profils de libération des microcapsules préparées selon comparatif 2.
Figure 7: Profils de libération in vitro des microcapsules préparées selon l'exemple 7 et comparaison avec les profils de libération des microcapsules préparées selon comparatif 3.
Figure 8: Profils de libération des microcapsules préparées selon les exemples 8, 9 et 10 à pH 1,4.
Figure 9: Profils de libération des microcapsules préparées selon les exemples 8, 9 et 10 mesurés pendant 2h à pH 1,4 puis à pH 6,8.
Figure 10: Profils de libération des microcapsules préparées selon les exemples 10, 11,12 et 13 à pH 1,4.
Figure 11: Profils de libération des microcapsules préparées selon l'exemple 14.
Figure 12: Profils de libération des microcapsules préparées selon l'exemple 15.
Figure 13: Profils de libération des microcapsules préparées selon l'exemple 16.
Figure 14: Profils de libération des microcapsules préparées selon l'exemple 17.

Les exemples ci-dessous portent sur les principes actifs suivants :

| Principe actif | Solubilité (g/l) |
|---|---|
| Spironolactone | 0,02 |
| Lansoprazole | 0,05 |
| Nitrofurantoïne | 0,3 |
| Amoxicilline trihydrate | 3,0 |
| Acyclovir | 10,0 |

Les exemples comparatif 1 (spironolactone), comparatif 2 (amoxicilline trihydrate), comparatif 3 (Nitrofurantoïne) et comparatif 4 (Carvedilol) illustrent des formulations à libération retardée et contrôlée du principe actif, obtenues selon le WO-A-03/03878. Cependant il serait avantageux de conserver la phase de latence tout en augmentant la vitesse de libération après la phase de latence, pour optimiser la biodisponibilité et l'efficacité du principe actif. Les microcapsules des exemples comparatifs 1 à 4 ne comportent pas d'agent gonflant.

Les exemples 5 (spironolactone), 6 (amoxicilline trihydrate) et 7 (Nitrofurantoïne) illustrent des formules selon l'invention.

Les exemples 8, 9 et 10 (acyclovir) montrent l'influence de la quantité d'agent gonflant présent dans les formules sur les cinétiques de libération à pH 1,4.

Les exemples 11, 12 et 13 (acyclovir) illustrent une sélection non exhaustive des agents gonflants pouvant être utilisés dans les formules selon l'invention.

L'exemple 14 (acyclovir) illustre la préparation de microcapsules associant une étape de granulation humide et une étape d'enrobage en lit d'air fluidisé.

L'exemple 15 (acyclovir) illustre la préparation de microcapsules associant une étape d'extrusion/sphéronisation et une étape d'enrobage en lit d'air fluidisé.

L'exemple 16 (acyclovir) illustre la préparation de microcapsules associant une étape de compactage et une étape d'enrobage en lit d'air fluidisé.

L'exemple 17 (acyclovir) illustre la préparation d'un médicament composé du mélange de différents types de micro-unités.

### Exemple comparatif 1 : Préparation de microcapsules de spironolactone ne contenant pas d'agent gonflant

### Etape 1:

432 g de Spironolactone et 48 g d'hydroxypropylcellulose de faible masse molaire (Klucel® EF /Hercules) sont dispersés dans 1120 g d'eau purifiée. La suspension est pulvérisée sur 720 g de microsphères neutres (Asahi-Kasei) dans un spray coater Glatt GPCG1.

### Etape 2:

43.2 g d'huile de coton hydrogénée (Penwest) et 64.8 g d'acide poly(methacrylique)(éthylacrylate) Eudragit® L100-55 (Röhm) sont dissous à chaud dans de l'isopropanol. La solution est pulvérisée sur 492 g de microparticules préparées précédemment.

Les microcapsules obtenues à l'issue de la deuxième étape ont été testées dans un dissolutest de type II conforme à la Pharmacopée Européenne 4ème Edition à 37°C et sous une agitation de 100 tours/min dans les milieux suivants :
▪ HCl à pH 1,4
▪ HCl à pH 1,4 pendant 2 heures puis milieu tampon KH₂PO₄ / NaOH à pH 6,8

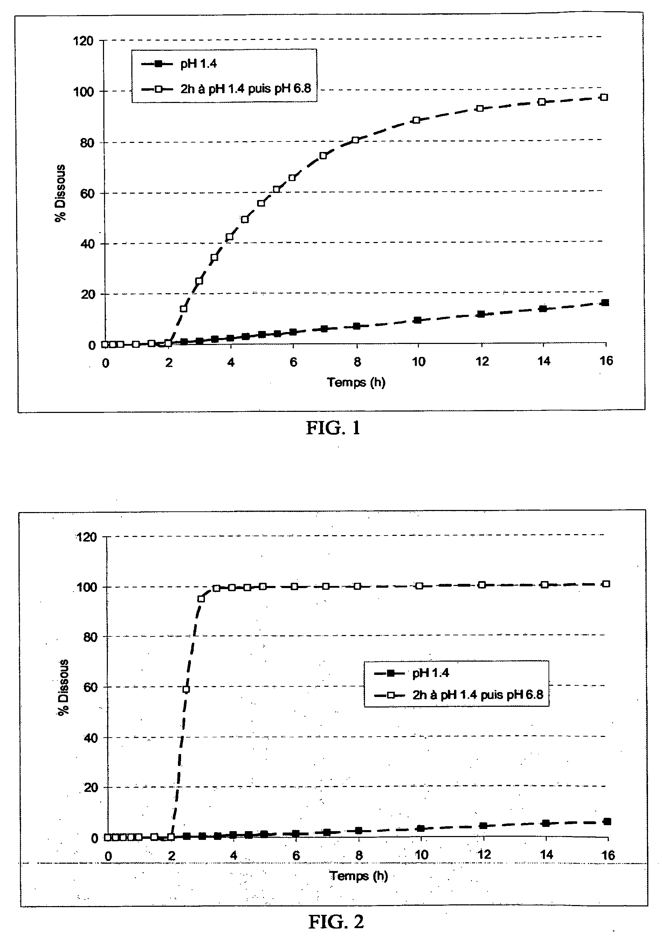

Les profils de dissolution sont présentés sur la Figure 1. On constate que :
- à pH 1,4 la libération du principe actif est lente après la période de latence d'environ 2 heures ;
- lors du passage de pH 1,4 à pH 6,8, la cinétique de libération accélère mais reste lente (il faut environ 8 heures pour libérer 80 % du principe actif).

Les nouvelles compositions selon l'invention permettent d'accélérer les profils de libération à pH 1,4 et à pH 6,8, tout en conservant la phase de latence à pH 1,4.

### Exemple comparatif 2 : Préparation de microcapsules d'amoxicilline trihydrate ne contenant pas d'agent gonflant

### Etape 1:

1620 g d'amoxicilline trihydrate et 180 g d'hydroxypropylcellulose de faible masse molaire (Klucel® EF (Hercules) sont dispersés dans 4200 g d'eau purifiée. La suspension est pulvérisée sur 200 g de microsphères neutres (Asahi-Kasei) dans un spray coater Glatt GPCG1.

### Etape 2:

120 g d'huile de coton hydrogénée (Penwest) et 180 g d'acide poly(methacrylique)(éthylacrylate) Acrycoat® L100D (NP Pharm) sont dissous à chaud dans de l'isopropanol. La solution est pulvérisée sur 700 g de microparticules préparées précédemment.

Les microcapsules obtenues à l'issue de la deuxième étape ont été testées dans un dissolutest de type II conforme à la Pharmacopée Européenne 4ème Edition à 37°C et sous une agitation de 100 tours/min dans les milieux suivants :
- HCl à pH 1,4
- HCl à pH 1,4 pendant 2 heures puis milieu tampon KH₂PO₄ / NaOH à pH 6,8

Les profils de dissolution sont présentés sur la figure 2. On constate que :
- à pH 1,4 la libération du principe actif est lente après la période de latence d'environ 4 heures ;
- lors du passage de pH 1,4 à pH 6,8, la cinétique de libération est rapide comme attendu.

Les nouvelles compositions selon l'invention permettent d'optimiser les profils de libération à pH 1,4 tout en maintenant une libération rapide à pH 6,8 et tout en conservant une phase de latence à pH 1,4.

### Exemple comparatif 3 : Préparation de microcapsules de Nitrofurantoïne ne contenant pas d'agent gonflant

### Etape 1:

640 g d'amoxicilline trihydrate et 160 g d'hydroxypropylcellulose de faible masse molaire (Klucel® EF/Hercules) sont dispersés dans 2400 g d'eau purifiée. La suspension est pulvérisée sur 200 g de microsphères neutres (Asahi-Kasei) dans un spray coater Glatt GPCG1.

### Etape 2:

40 g d'huile de coton hydrogénée (Penwest), 5 g de dibutylsébaçate (Morflex) et 55 g d'acide (polymethacrylique)(méthylmethacrylate) Eudragit® L100 (Röhm) sont dissous à chaud dans de l'isopropanol. La solution est pulvérisée sur 900 g de microparticules préparées précédemment.

Les microcapsules obtenues à l'issue de la deuxième étape ont été testées dans un dissolutest de type II conforme à la Pharmacopée Européenne 4ème Edition, à 37°C et sous une agitation de 100 tours/min dans les milieux suivants :
- HCl à pH 1,4
- HCl à pH 1,4 pendant 2 heures puis milieu tampon KH₂PO₄ / NaOH à pH 6,8

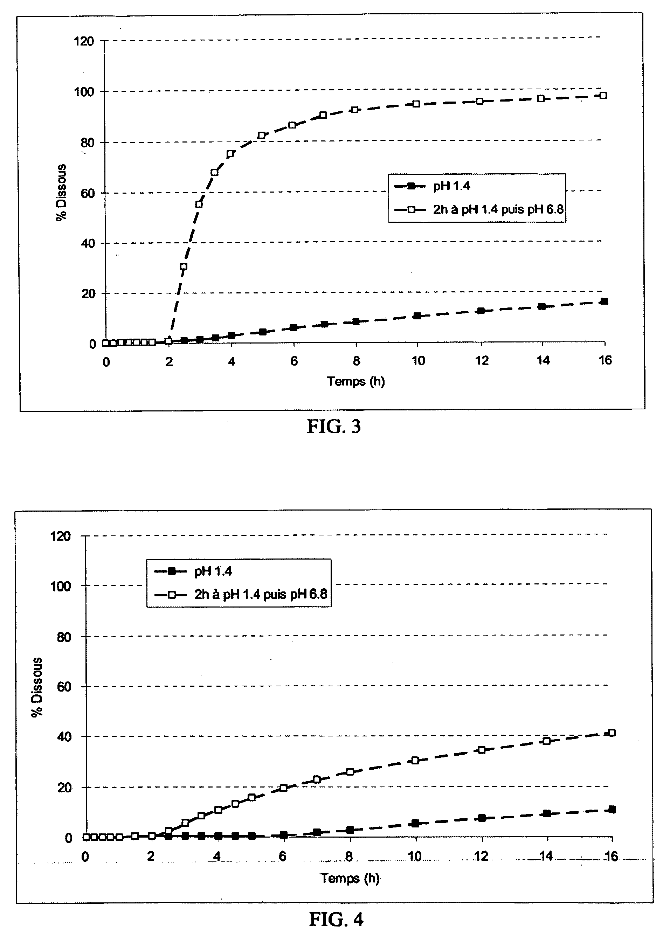

Les profils de dissolution sont présentés sur la Figure 3. On constate que :
- à pH 1,4 la libération du principe actif est lente après la période de latence d'environ 2 heures ;
- lors du passage de pH 1,4 à pH 6,8, la cinétique de libération est rapide comme attendu.

Les nouvelles compositions selon l'invention permettent d'optimiser les profils de libération à pH 1,4 tout en maintenant une libération rapide à pH 6,8 et tout en conservant une phase de latence à pH 1,4.

### Exemple comparatif 4 : Préparation de microcapsules de Carvedilol phosphate ne contenant pas d'agent gonflant

1120 g de Carvédilol phosphate et 280 g de Plasdone K29/32® (ISP) sont dispersés dans 1120 g d'eau purifiée. La suspension est pulvérisée sur 600 g de microsphères neutres (Asahi-Kasei) dans un spray coater Glatt GPCG1.

100 g d'huile de coton hydrogénée (Penwest) et 150 g d'Eudragit® L100-55 (Röhm) sont dissous à chaud dans de l'isopropanol. La solution est pulvérisée sur 750 g de microparticules préparées précédemment.

Les microcapsules obtenues à l'issue de la deuxième étape ont été testées dans un dissolutest de type II conforme à la Pharmacopée à 37°C et sous une agitation de 100 tours/min dans les milieux suivants :
- HCl à pH 1,4
- HCl à pH 1,4 pendant 2 heures puis milieu tampon KH₂PO₄ / NaOH à pH 6,8

Les profils de dissolution sont présentés sur la figure 4 annexée. On constate que :
- à pH 1.4 la libération du principe actif est lente après la période de latence d'environ 2 heures ;
- lors du passage de pH 1.4 à pH 6.8, la cinétique de libération accélère mais reste lente (à 16 heures il y a seulement 40% du principe actif qui est libéré).

### Exemple 5: Préparation de microcapsules de spironolactone selon l'invention

### Etape 1:

216 g de Spironolactone, 72 g d'hydroxypropylcellulose de faible masse molaire (Klucel® EF /Hercules), 72 g de PEG-40 huile de ricin hydrogénée (Cremophor RH 40 / BASF) et 360 g de crospovidone (Kollidon CL / BASF) sont dispersés dans 1120 g d'eau purifiée. La suspension est pulvérisée sur 720 g de microsphères neutres (Asahi-Kasei) dans un spray coater Glatt GPCG1.

### Etape 2:

43.2 g d'huile de coton hydrogénée (Penwest) et 64.8 g d'acide poly(methacrylique)(éthylacrylate) Eudragit® L100-55 (Röhm) sont dissous à chaud dans de l'isopropanol. La solution est pulvérisée sur 492 g de microparticules préparées précédemment.

Les microcapsules obtenues à l'issue de la deuxième étape ont été testées dans un dissolutest de type II conforme à la Pharmacopée Européenne 4ème Edition, à 37°C et sous une agitation de 100 tours/min dans les milieux suivants :
- HCl à pH 1,4
- HCl à pH 1,4 pendant 2 heures puis milieu tampon KH₂PO₄ / NaOH à pH 6,8

Les profils de dissolution de l'exemple 5 et de l'exemple comparatif 1 sont présentés sur la Figure 4. On constate que :
- à pH 1.4 environ 60% du principe actif est libéré après une période de latence d'environ 1,5 heures ;
- lors du passage de pH 1,4 à pH 6,8, la cinétique de libération est rapide.

### Exemple 6: Préparation de microcapsules d'amoxicilline trihydrate selon l'invention

### Etape 1:

630 g d'Amoxicilline trihydrate, 90 g de povidone (plasdone® K29/32 (ISP)) et 180 g de crospovidone (Polyplasdone® /ISP) sont dispersés dans 2100 g de mélange isopropanol/eau (70/30 m/m). La solution est pulvérisée sur 100 g de microsphères neutres (Asahi-Kasei) dans un spray coater Glatt® GPCG1.

### Etape 2:

120 g d'huile de coton hydrogénée (Abitec) et 160 g d'acide poly(methacrylique)(éthylacrylate) Kollicoat®MAE 100P (BASF) sont dissous à chaud dans de l'isopropanol. La solution est pulvérisée sur 700 g de microparticules préparées précédemment.

Les microcapsules obtenues à l'issue de la deuxième étape ont été testées dans un dissolutest de type II conforme à la Pharmacopée Européenne 4ème Edition, à 37°C et sous une agitation de 100 tours/min dans les milieux suivants :
- HCl à pH 1,4
- HCl à pH 1,4 pendant 2 heures puis milieu tampon KH₂PO₄ / NaOH à pH 6,8

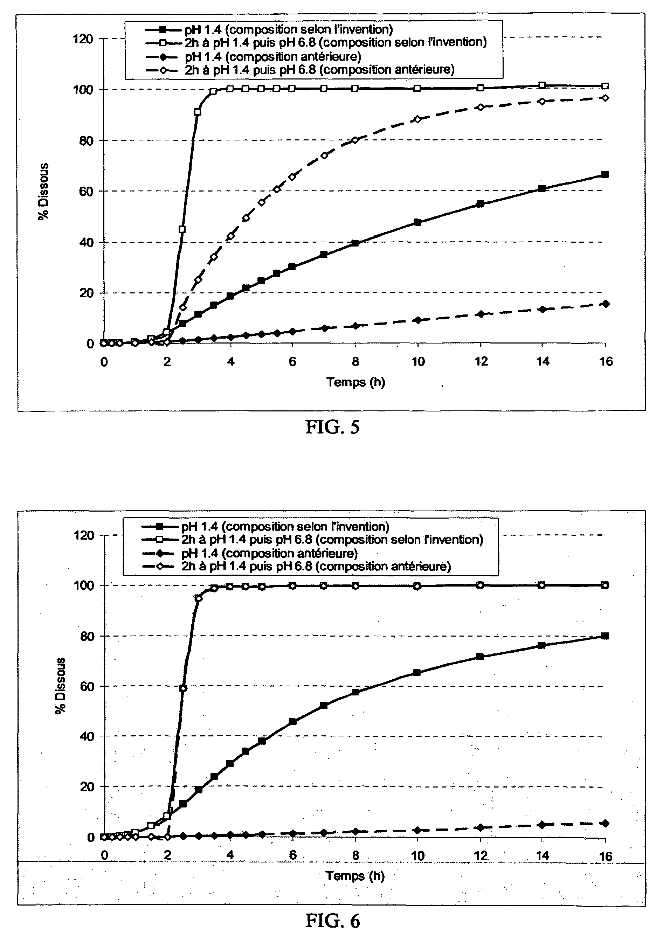

Les profils de dissolution de l'exemple 6 et de l'exemple comparatif 2 sont présentés sur la Figure 6. On constate qu'avec la composition selon l'invention :
- la libération du principe actif à pH 1,4 a été accélérée (garantissant le déclenchement du système au bout d'un temps donné et la libération d'une quantité suffisante d'actif. Cette libération se faisant sur des temps compatibles avec les temps d'absorption des principes actifs dans l'organisme) ;
- lors du passage de pH 1,4 à pH 6,8, une cinétique de libération rapide est maintenue.

### Exemple 7: Préparation de microcapsules de Nitrofurantoïne selon l'invention

### Etape 1:

400 g de Nitrofurantoïne, 200 g de povidone (plasdone® K29/32 /ISP), 50 g de PEG-40 huile de ricin hydrognée (BASF) et 350 g de crospovidone (Polyplasdone® /ISP) sont mis en suspension dans 2500 g d'eau purifiée. La solution est pulvérisée sur 1000 g de microsphères neutres (Asahi-Kasei) dans un spray coater Glatt® GPCG1.

### Etape 2:

120 g d'huile de coton hydrogénée (Abitec) et 160 g d'acide poly(methacrylique)(éthylacrylate) Acrycoat® L100D (NP Pharm) sont dissous à chaud dans de l'isopropanol. La solution est pulvérisée sur 700 g de microparticules préparées précédemment.

Les microcapsules obtenues à l'issue de la deuxième étape ont été testées dans un dissolutest de type II conforme à la Pharmacopée Européenne 4ème Edition à 37°C et sous une agitation de 100 tours/min dans les milieux suivants :
- HCl à pH 1,4
- HCl à pH 1,4 pendant 2 heures puis milieu tampon KH₂PO₄ / NaOH à pH 6,8

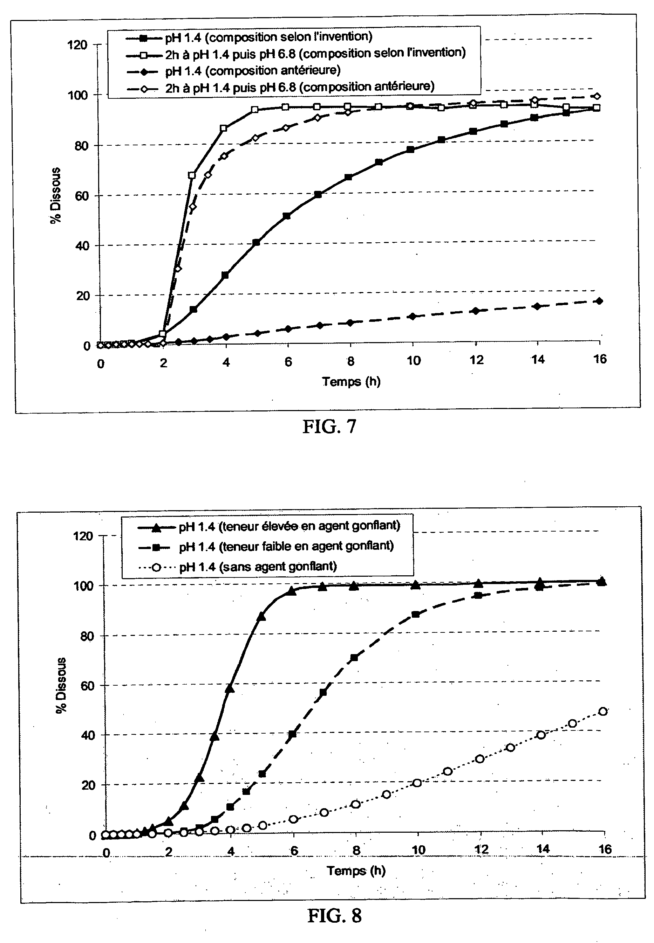

Les profils de dissolution de l'exemple 7 et de l'exemple comparatif 3 sont présentés sur la Figure 7. On constate qu'avec la composition selon l'invention :
- la libération du principe actif à pH 1,4 a été accélérée (garantissant le déclenchement du système au bout d'un temps donné et la libération d'une quantité suffisante d'actif. Cette libération se faisant sur des temps compatibles avec les temps d'absorption des principes actifs dans l'organisme) ;
- lors du passage de pH 1,4 à pH 6,8, une cinétique de libération rapide est maintenue.

### Exemple comparatif 8: Préparation de microcapsules d'Acyclovir ne contenant pas d'agent agent gonflant

### Etape 1:

75 g d'Acyclovir et 75 g de Povidone (Plasdone® K29/32 / ISP) sont dissous dans 833 g d'isopropanol. La solution est pulvérisée sur 850 g de microsphères neutres (NP Pharm). dans un spray coater Glatt® GPCG3.

### Etape 2:

93.3 g d'huile de soja hydrogénée (Abitec) et 140 g d'acide (polymethacrylique)(méthylinethacrylate) Eudragit® L100 (Röhm) sont dissous à chaud dans de l'isopropanol. La solution est pulvérisée sur 700 g de microparticules préparées précédemment.

### Exemple 9: Préparation de microcapsules d'Acyclovir contenant une faible quantité d'agent gonflant (crospovidone®)

### Etape 1:

375 g d'Acyclovir, 50 g d'hydroxypropylcellulose de faible masse molaire (Klucel® EF (Hercules) et 75 g de crospovidone (Polyplasdone® /ISP) sont mis en suspension dans 1200 g d'eau purifiée. La solution est pulvérisée sur 500 g de microsphères neutres (NP Pharm). dans un spray coater Glatt® GPCG3.

### Etape 2:

100 g d'huile de coton hydrogénée (Penwest) et 150 g d'acide poly(methacrylique)(éthylacrylate) Eudragit® L100-55 (Röhm) sont dissous à chaud dans de l'ethanol. La solution est pulvérisée sur 750 g de microparticules préparées précédemment.

### Exemple 10 : Préparation de microcapsules d'Acyclovir contenant une quantité plus élevée d'agent gonflant (crospovidone®)

### Etape 1:

300 g d'Acyclovir, 50 g d'hydroxypropylcellulose de faible masse molaire Klucel® EF (Hercules) et 150 g de crospovidone (Polyplasdone® / ISP) sont mis en suspension dans 1200 g d'eau purifiée. La solution est pulvérisée sur 500 g de microsphères neutres (NP Pharm). dans un spray coater Glatt® GPCG3.

### Etape 2:

100 g d'huile de coton hydrogénée (Penwest) et 150 g d'acide poly(methacrylique)(éthylacrylate) Eudragit® L100-55 (Röhm) sont dissous à chaud dans de l'éthanol. La solution est pulvérisée sur 750 g de microparticules préparées précédemment.

Les microcapsules obtenues à l'issue de la deuxième étape dans les exemples comparatif 8, 9 & 10, ont été testées dans un dissolutest de type II conforme à la Pharmacopée Européenne 4ème Edition à 37°C et sous une agitation de 100 tours/min dans les milieux suivants:
- HCl à pH 1,4
- HCl à pH 1,4 pendant 2 heures puis milieu tampon KH₂PO₄ / NaOH à pH 6,8

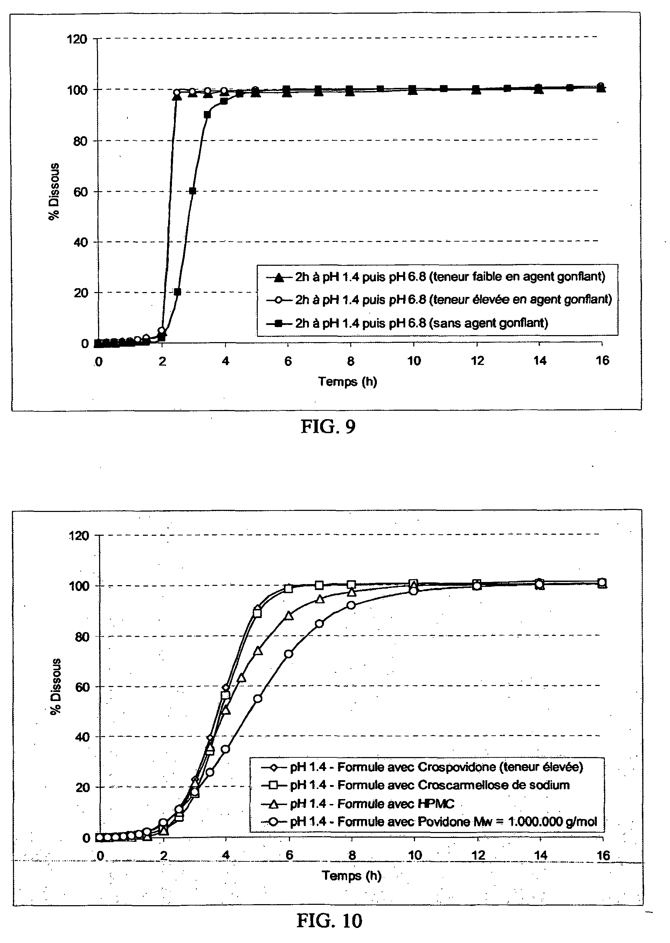

Les profils de dissolution des exemples 8, 9 & 10 sont présentés sur les Figures 8 & 9. On constate que :
- Une large gamme de cinétique peut être obtenue à pH 1.4 en fonction de la quantité d'agent gonflant incorporé dans la formulation ;
- La libération à pH 6,8 reste rapide quelque soit la composition considérée.

### Exemple 11: Préparation de microcapsules d'Acyclovir contenant un agent gonflant (Croscarmellose de sodium)

### Etape 1:

300 g d'Acyclovir, 50 g d'hydroxypropylcellulose de faible masse molaire Klucel® EF (Hercules) et 150 g de Croscarmellose de sodium (Ac-Di-Sol® /FMC) sont mis en suspension dans 1200 g d'eau purifiée. La solution est pulvérisée sur 500 g de microsphères neutres (NP Pharm) dans un spray coater Glatt® GPCG1.

### Etape 2:

100 g d'huile de coton hydrogénée (Penwest), et 100 g d'acide poly(methacrylique)(éthylacrylate) Eudragit® L100-55 (Röhm) sont dissous à chaud dans de l'éthanol. La solution est pulvérisée sur 750 g de microparticules préparées précédemment.

### Exemple 12 : Préparation de microcapsules d'Acyclovir contenant un agent gonflant (Hydroxypropylméthylcellulose)

### Etape 1:

300 g d'Acyclovir, 50 g d'hydroxypropylcellulose de faible masse molaire Klucel® EF (Hercules) et 150 g d'hydroxypropylméthylcellulose (Pharmacoat 615/ Shin-Etsu) sont mis en suspension dans 1200 g d'eau purifiée. La solution est pulvérisée sur 500 g de microsphères neutres (NP Pharm). dans un spray coater Glatt® GPCG1.

100 g d'huile de coton hydrogénée (Penwest), 100 g d'acide poly(methacrylique)(éthylacrylate) Eudragit® L100-55 (Röhm) et 50 g d'acide poly(methacrylique)(methylmethacrylate) Eudragit® S100 (Rôhm) sont dissous à chaud dans de l'éthanol. La solution est pulvérisée sur 750 g de microparticules préparées précédemment.

### Exemple 13 : Préparation de microcapsules d'Acyclovir contenant un agent gonflant (povidone de masse molaire Mw=1000000 g/mol)

### Etape 1:

350 g d'Acyclovir, 50 g d'hydroxypropylcellulose de faible masse molaire (Klucel® EF (Hercules) et 100 g de povidone de haute masse molaire (Kollidon®90 (BASF) sont mis en suspension dans 1200 g d'eau purifiée. La solution est pulvérisée sur 500 g de microsphères neutres (NP Pharm) dans un spray coater Glatt® GPCG1.

### Etape 2:

100 g d'huile de coton hydrogénée (Penwest), 50 g d'acide poly(methacrylique)(éthylacrylate) Eudragit® L100-55 (Röhm) et 100 d'acide poly(methacrylique)(methylmethacrylate) Eudragit® S100 (Röhm) sont dissous à chaud dans de l'ethanol. La solution est pulvérisée sur 750 g de microparticules préparées précédemment.

Les microcapsules obtenues à l'issue de la deuxième étape des exemples 10, 11, 12 et 13 ont été testées dans un dissolutest de type II conforme à la Pharmacopée Européenne 4ème Edition à 37°C et sous une agitation de 100 tours/min à pH 1,4.

Les profils de dissolution sont présentés sur la Figure 10.

### Exemple 14 : Préparation de microcapsules d'Acyclovir contenant un agent gonflant (granulation + spray-coating)

### Etape 1:

700 g d'acyclovir, 50 g de Povidone (Plasdone® /ISP) et 250 g de Crospovidone (Polyplasdone®/ ISP) sont préalablement mélangés à sec dans un granulateur de laboratoire (Lodige) pendant 5 minutes. Ce mélange pulvérulent est ensuite granulé à l'eau (200 g). Les granulés sont séchés à 40°C en étuve ventilée, puis calibrés sur grille de 500 µm. On sélectionne par tamisage la fraction 200-500 µm.

### Etape 2:

100 g d'huile de palme hydrogénée (Huls), 100 g d'acide poly(methacrylique)(éthylacrylate) Acrycoat L100D et 50 g d'acide poly-(methacrylique)(methylmethacrylate) Acrycoat® S100 (NP Pharm) sont dissous à chaud dans de l'isopropanol. La solution est pulvérisée sur 750 g de microparticules préparées précédemment.

Les microcapsules obtenues à l'issue de la deuxième étape de l'exemple 13 ont été testées dans un dissolutest de type II conforme à la Pharmacopée Européenne 4ème Edition à 37°C et sous une agitation de 100 tours/min dans les milieux suivants :
- HCl à pH 1,4
- HCl à pH 1,4 pendant 2 heures puis milieu tampon KH₂PO₄ / NaOH à pH 6,8

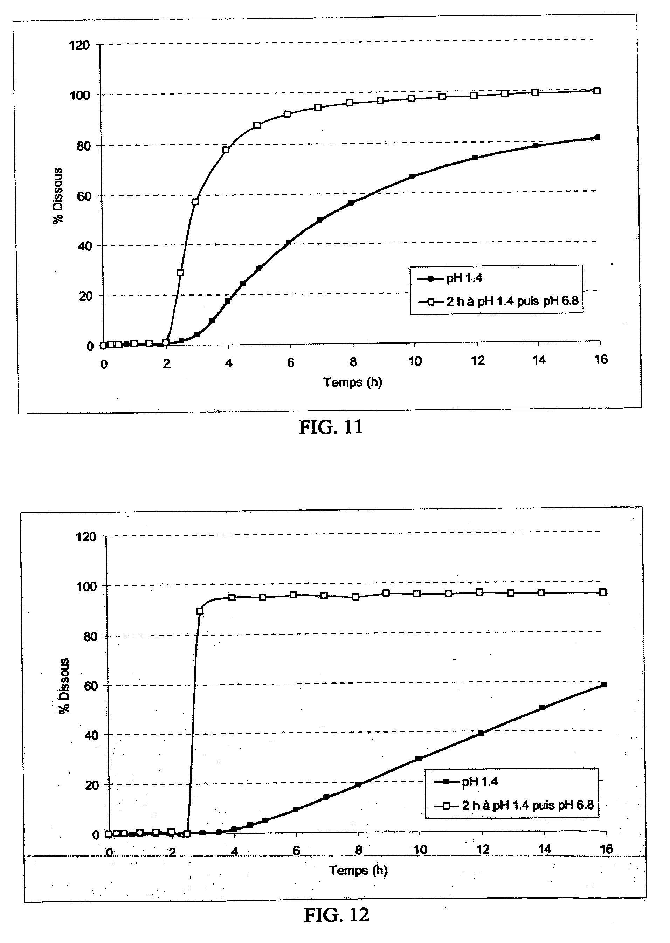

Les profils de dissolution sont présentés sur la Figure 11.

### Exemple 15 : Préparation de microcapsules d'Acyclovir contenant un agent gonflant (extrusion/sphéronisation + spray-coating)

### Etape 1:

700 g d'acyclovir, 50 g de Povidone (Plasdone® /ISP) et 250 g de Crospovidone (Kollidon ®CL / BASF) sont préalablement mélangés avec 150 g d'eau dans un mélangeur de laboratoire (Kitchen-Aid) pendant 5 minutes. Ce mélange pâteux est extrudé au travers d'une grille de 0.5 mm à l'aide d'un extrudeur 20 (Caleva). Les filaments obtenus sont ensuite sphéronisés à l'aide d'un sphéroniseur 250 (Caleva). Les particules obtenues sont séchées à 40°C en lit d'air fluidisé. On sélectionne par tamisage la fraction 300-700 µm.

### Etape 2:

100 g d'huile de palme hydrogénée (Huls), 100 g d'acide poly(methacrylique)(éthylacrylate) Acrycoat® L100D et 50 g d'acide poly(methacrylique)(methylmethacrylate) Acrycoat® S100 (NP Pharm) sont dissous à chaud dans de l'isopropanol. La solution est pulvérisée sur 750 g de microparticules préparées précédemment.

Les microcapsules obtenues à l'issue de la deuxième étape de l'exemple 14 ont été testées dans un dissolutest de type II conforme à la Pharmacopée 4ème Edition à 37°C et sous une agitation de 100 tours/min dans les milieux suivants :
- HCl à pH 1,4
- HCl à pH 1,4 pendant 2 heures puis milieu tampon KH₂PO₄ / NaOH à pH 6,8

Les profils de dissolution sont présentés sur la Figure 12.

### Exemple 16 : Préparation de microcapsules d'Acyclovir contenant un agent gonflant (compactage + spray-coating)

### Etape 1:

590 g d'acyclovir, 10 g de stéarate de Magnésium et 400 g de crospovidone sont mélangés à l'aide d'un mélangeur de laboratoire (type Kitchen-Aid) pendant 5 minutes. Ce mélange est ensuite compacté l'aide d'un compacteur de laboratoire Alexenderwerk WP120. Le produit obtenu est ensuite granulé à l'aide d'un granulateur oscillant Erweka équipé d'une grille de 500 µm. On sélectionne par tamisage la fraction 100-500 µm du produit obtenu.

### Etape 2:

100 g d'huile de palme hydrogénée (Huls), 100 g d'acide poly(methacrylique)(éthylacrylate) Acrycoat L100D et 50 g d'acide poly(methacrylique)-(methylmethacrylate) Acrycoat® S100 (NP Pharm) sont dissous à chaud dans de l'isopropanol. La solution est pulvérisée sur 750 g de microparticules préparées précédemment.

Les microcapsules obtenues à l'issue de la deuxième étape de l'exemple 14 ont été testées dans un dissolutest de type II conforme à la Pharmacopée 4ème Edition à 37°C et sous une agitation de 100 tours/min dans les milieux suivants :
- HCl à pH 1,4
- HCl à pH 1,4 pendant 2 heures puis milieu tampon KH₂PO₄ / NaOH à pH 6,8

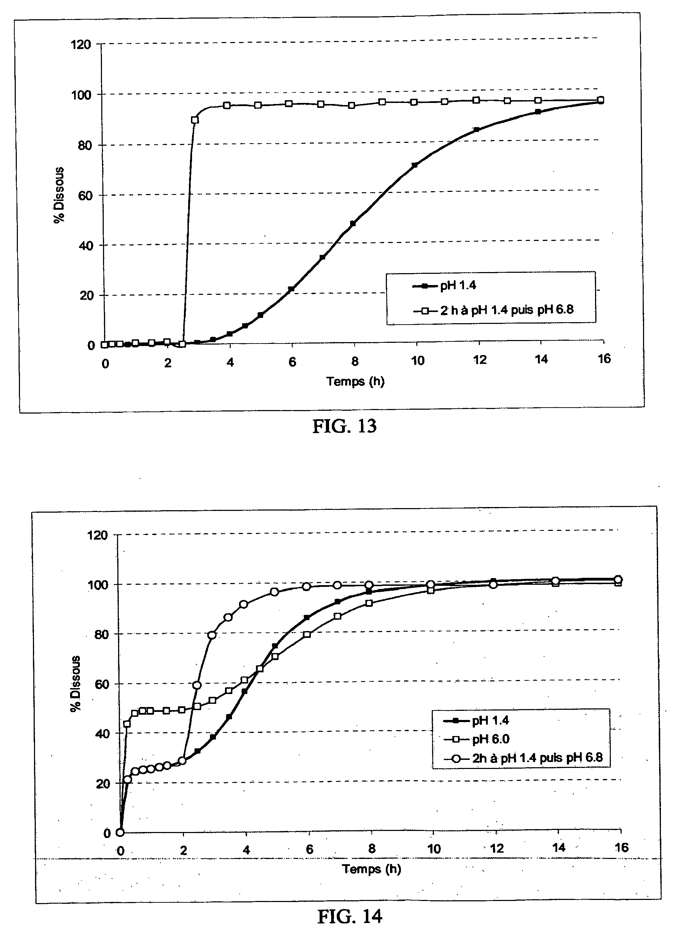

Les profils de dissolution sont présentés sur la Figure 13.

### Exemple 17 : Mélange de micro-unités ayant différents profils de libération

On prépare de différentes micro-unités d'acyclovir dans lesquelles:
- 25% en poids de l'acyclovir est présenté sous la forme de micro-unités à libération immédiate telle qu'obtenues à l'issue de la première étape de l'exemple 12,
- 25% de l'acyclovir est présenté sous la forme de micro-unités à libération retardée et prolongée telle qu'obtenues à l'issue de la deuxième étape de l'exemple 10 et
- 50% de l'acyclovir est présenté sous la forme de microcapsules à libération retardée et prolongée telle qu'obtenues à l'issue de la deuxième étape de l'exemple 12.

Les microcapsules de l'exemple n°10 commencent à libérer rapidement leur contenu au-délà de pH ≥ 5.5 (utilisation d'Eudragit® L100-55).

Les microcapsules de l'exemple n°12 commencent à libérer rapidement leur contenu au-delà de pH > 6.5 (utilisation de 67% Eudragit® L100-55 et 33% Eudragit S100).

Les profils sont présentés figure 14 qui montrent que l'on obtient différentes phases de libération, ce qui optimiser la libération d'un principe actif devant sa fenêtre d'absorption.

## Revendications

1. Médicament oral comprenant une pluralité de microcapsules à libération modifiée de principe(s) actif(s), au moins une partie desdites microcapsules étant individuellement constituée d'une microparticule comprenant au moins un principe actif, en particulier au moins un principe actif peu soluble à l'exclusion du carvédilol, enrobée d'au moins un revêtement permettant la libération modifiée du(des) principe(s) actif(s), ladite libération étant régie par deux mécanismes distincts de déclenchement, l'un basé sur une variation de pH et l'autre permettant la libération du(ou des) principe(s) actif(s), au bout d'un temps prédéterminé de résidence dans l'estomac,
ledit revêtement conférant également aux microcapsules un comportement de dissolution in vitro tel que:
- à pH constant 1,4, le profil de dissolution comporte une phase de latence de durée inférieure ou égale à 7 heures, de préférence inférieure ou égale à 5 heures, et, plus préférentiellement encore comprise entre 1 à 5 heures;
- le passage de pH 1,4 à pH 7,0, conduit à une phase de libération débutant sans temps de latence;
**caractérisé**
- **en ce qu'**au moins une partie desdites microcapsules comprend au moins un agent gonflant,
- et **en ce que** la fraction en poids du(ou des) principe(s) actif(s) libéré(s) pendant la phase de latence est inférieure ou égale à 15 % en poids par heure, de préférence inférieure ou égale à 10 % en poids par heure, et, plus préférentiellement encore inférieure ou égale à 5 % en poids par heure.

2. Médicament selon la revendication 1, **caractérisé en ce que** l'agent gonflant comprend au moins un composé, pharmaceutiquement acceptable, hydrophile et présentant un taux de gonflement dans l'eau à 25°C, supérieur ou égal à 10 % en poids, de préférence supérieur ou égal à 15 % en poids et plus préférentiellement encore supérieur ou égal à 20%.

3. Médicament selon la revendication 1, **caractérisé en ce que** l'agent gonflant est choisi parmi ceux permettant aux microcapsules de libérer au moins 50% en poids du principe actif, après 16 h à pH 1,4, dans un test de dissolution in vitro.

4. Médicament selon la revendication 1, **caractérisé en ce que** l'agent gonflant est sous forme de microparticules, de diamètre moyen compris entre 5 et 200 µm et, de préférence, entre 10 et 50 µm.

5. Médicament selon la revendication 1 **caractérisé en ce que** la concentration (Cd) en agent gonflant est comprise entre 3% et 40% en poids par rapport à la masse totale des microcapsules, de préférence entre 4% et 30% et plus préférentiellement encore entre 5% et 25%.

6. Médicament selon la revendication 1, **caractérisé en ce que** l'agent gonflant est choisi dans le groupe de produits suivants:
o les polyvinylpyrrolidones réticulées (par exemple la polyplasdone ou la crospovidone),
o les carboxyalkylcelluloses réticulées: les carboxyméthylcelluloses réticulées (par exemple la croscarmellose),
o ainsi que les polymères hydrophiles de haute masse molaire (supérieure ou égale à 100. 000 D) tels que:
▪ les polyvinylpyrrolidones,
▪ les polyalkylènes oxydes (par exemple de polyéthylène oxyde ou de polypropylène oxyde),
▪ les (hydroxy)(alkyl) celluloses (par exemple l' hydroxypropylcellulose, l'hydroxypropylméthylcellulose),
▪ les carboxyalkylcelluloses (par exempte la carboxyméthylcellulose),
▪ les celluloses (poudre ou microcristalline),
▪ les amidons modifiés (par exemple avec du glycolate de sodium),
▪ les amidons natifs (par exemple de maïs, de blé ou de pomme de terre),
▪ l'alginate de sodium,
▪ la polacriline de potassium,
▪ et leurs mélanges.

7. Médicament selon l'une des revendications précédentes, **caractérisé en ce qu'**il comprend au moins un agent mouillant, de préférence choisi dans le groupe de produits suivants:
∘ les tensioactifs anioniques, de préférence dans le sous-groupe des sels alcalins ou alcalinoterreux des acides gras, l'acide stéarique et oléique étant préférés,
∘ les tensioactifs non ioniques, de préférence dans le sous-groupe suivant:
▪ les huiles polyoxyéthylénées, de préférence l'huile de ricin hydrogénée polyoxyéthylénée,
▪ les copolymères polyoxyéthylène-polyoxypropylène,
▪ les esters de sorbitan polyoxyéthylénés,
▪ les dérivés de l'huile de ricin polyoxyéthylénés,
▪ les stéarates, de préférence de calcium, de magnésium, d'aluminium ou de zinc,
▪ les stéarylfurmarates, de préférence de sodium,
▪ le béhénate de glycérol,
▪ et leurs mélanges.

8. Médicament selon l'une des revendications précédentes **caractérisé en ce que** l'agent gonflant est contenu dans la microparticule de principe actif.

9. Médicament selon l'une des revendications précédentes **caractérisé en ce que** l'agent mouillant est contenu dans la microparticule de principe actif.

10. Médicament selon l'une des revendications précédentes, **caractérisé en ce que** les microcapsules de principe(s) actif(s) qu'il comprend, sont aptes à libérer au moins 80% en poids du (ou des) principe(s) actif(s), après 12 h à pH= 7,0, dans un test de dissolution in vitro.

11. Médicament selon l'une des revendications précédentes, **caractérisé en ce qu'**au moins une partie desdites microcapsules à libération modifiée de principe actif comporte:
o un coeur neutre,
o au moins une couche active comprenant le (ou les) principe(s) actif(s)et enrobant le coeur neutre, et
o au moins un revêtement permettant la libération modifiée du principe actif.

12. Médicament selon l'une des revendications précédentes, **caractérisé en ce que**
o le revêtement permettant la libération modifiée du (ou des) principe(s) actif(s) comprend un matériau composite
▪ comportant:
• au moins un polymère hydrophile A porteur de groupements ionisés à pH neutre,
• au moins un composé hydrophobe B;
▪ représentant une fraction massique (% poids par rapport à la masse totale des microcapsules) inférieur ou égal à 40 %;
∘ les microcapsules ont un diamètre moyen inférieur à 2000 µm.

13. Médicament selon la revendication 12, **caractérisé en ce que** le matériau composite AB du revêtement permettant la libération modifiée du principe actif peu soluble, est tel que:
∘ le ratio pondéral B/A, est compris entre 0,2 et 1,5, de préférence entre 0,5 et 1,0,
∘ et le composé B hydrophobe est sélectionné parmi les produits cristallisés à l'état solide et ayant une température de fusion TfB supérieur ou égal à 40°C, de préférence TfB supérieur ou égal à 50°C, et plus préférentiellement compris entre 40°C et 90°C.

14. Médicament selon l'une des revendications 12 et 13, **caractérisé en ce que** le polymère hydrophile A est choisi parmi: les polymères d'acide (méth)acrylique, les polymères d'alkyl(méth)acrylate, les copolymères d'acide (méth)acrylique et d'alkyl(méth)acrylate, les dérivés de celluloses, les phtalates de cellulose, l'acétate phtalate de cellulose, les succinates de cellulose, l'acétate succinate de cellulose, les phtalates d'hydroxypropylméthylcellulose, les succinates d'hydroxypropylméthylcellulose et leurs mélanges,

15. Médicament selon l'une des revendications 12 à 14, **caractérisé en ce que** le composé B est choisi parmi le groupe de produits suivants: les huiles végétales hydrogénées, les cires végétales, la cire jaune, la cire blanche, la cire microcristalline, la lanoline, les matières grasses laitières anhydres, la matière grasse dure ou matière grasse utile comme bases de suppositoires, les macrogolglycérides lauryliques, l'alcool cétylique, le diisostéarate de polyglycérol, les monoesters, les diesters et les triesters du glycérol et d'au moins un acide gras, les mélanges de monoesters, de diesters et de triesters de glycérol et d'au moins un acide gras, l'éthylène glycol monostéarate, le diéthylène glycol monostéarate et leurs mélanges.

16. Médicament selon la revendication 15, **caractérisé en ce que** le composé B est choisi parmi le groupe de produits suivants: huile hydrogénée de graines de coton, huile hydrogénée de graines de soja, huile de palme hydrogénée, béhénate de glycérol, huile de ricin hydrogénée, tristéarine, tripalmitine, trimyristine, Oméga 3 et tout mélange d'entre eux.

17. Médicament selon l'une quelconque des revendications 12 à 16, **caractérisé en ce que** le revêtement des microcapsules à libération modifiée de principe actif comprend une seule pellicule d'enrobage comportant le composite AB.

18. Médicament oral comprenant une pluralité de microcapsules à libération modifiée de principe(s) actif(s), au moins une partie desdites microcapsules étant individuellement constituée d'une microparticule comprenant au moins un principe actif en particulier au moins un principe actif peu soluble à l'exclusion du carvédilol enrobée d'au moins un revêtement permettant la libération modifiée du(des) principe(s) actif(s), ladite libération étant régie par deux mécanismes distincts de déclenchement, l'un basé sur une variation de pH et l'autre permettant la libération du(ou des) principe(s) actif(s), au bout d'un temps prédéterminé de résidence dans l'estomac,
ledit revêtement:
∘ conférant également aux microcapsules un comportement de dissolution in vitro tel que:
- à pH constant 1,4, le profil de dissolution comporte une phase de latence de durée inférieure ou égale à 7 heures, de préférence inférieure ou égale à 5 heures, et, plus préférentiellement encore comprise entre 1 à 5 heures;
- le passage de pH 1,4 à pH 7,0, conduit à une phase de libération débutant sans temps de latence;
∘ et comprenant un matériau composite comportant au moins un polymère hydrophile A porteur de groupements ionisés à pH neutre et au moins un composé hydrophobe B;
**caractérisé en ce qu'**au moins une partie desdites microcapsules comprend au moins un auxiliaire de libération apte à augmenter la perméabilité du revêtement permettant la libération modifiée du(ou des) principe(s) actif(s), et **en ce que** la fraction en poids du(ou des) principe(s) actif(s) libéré(s) pendant la phase de latence est inférieure ou égale à 15 % en poids par heure, de préférence inférieure ou égale à 10 % en poids par heure, et, plus préférentiellement encore inférieure ou égale à 5 % en poids par heure.

19. Médicament selon la revendication 18 **caractérisé en ce que** l'auxiliaire de libération est constitué par au moins un agent gonflant.

20. Médicament selon la revendication 18 ou 19 **caractérisé en ce que** le revêtement des microcapsules leur confère un comportement de dissolution in vitro tel qu'au moins 50% en poids du(ou des) principe(s) actif(s) sont libérés après 16h à pH 1,4.

21. Médicament en particulier mais non limitativement selon l'une des revendications 1 à 20, **caractérisé en ce qu'**il comprend un mélange de différentes populations de micro-unités contenant du(ou des) principe(s) actif(s) à l'exclusion du carvédilol, ces populations différant entre elles par leurs profils de dissolution in vitro respectifs, pour au moins une valeur de pH comprise entre 1,4 et 7,4.

22. Médicament selon la revendication 20 et au moins l'une des autres revendications précédentes, **caractérisé en ce que** les micro-unités sont des microcapsules à libération modifiée de principe(s) actif(s) et éventuellement des micro-unités à libération immédiate de principe(s) actif(s).

23. Médicament selon la revendication 21, **caractérisé en ce que** les populations de microcapsules à libération modifiée de principe actif diffèrent par leurs pH de déclenchement respectifs.

24. Médicament selon la revendication 21, **caractérisé en ce que** les populations de microcapsules à libération modifiée de principe actif diffèrent par leurs temps de déclenchement respectifs.

25. Médicament selon la revendication 21, **caractérisé en ce qu'**il comprend:
i. au moins une population de micro-unités contenant du principe actif à libération immédiate;
ii. au moins une population P1 de microcapsules à libération modifiée de(s) principe(s) actif(s), et
iii. au moins une population P2 de microcapsules à libération modifiée de(s) principe(s) actif(s);
et **en ce que** les pH de déclenchement respectifs de P1 et de P2 diffèrent d'au moins 0,5 unité de pH, de préférence d'au moins 0,8 unités de pH, et, plus préférentiellement encore, d'au moins 0, 9 unité de pH.

26. Médicament selon la revendication 21, **caractérisé en ce que** les pH de déclenchement respectifs des différentes populations de microcapsules à libération modifiée de principe(s) actif(s), sont compris 5 et 7.

27. Médicament selon la revendication 21, **caractérisé en ce qu'**il comprend:
i. au moins une population de micro-unités contenant du (des) principe(s) actif(s) à libération immédiate.
ii. au moins une population P1' de micro-unités contenant du (des) principe(s) actif(s) formées par des microcapsules à libération modifiée du (des) principe(s) actif(s), dont le pH de déclenchement est égal à 5,5, et
iii. au moins une population P2' de micro-unités contenant du (des) principe(s) actif(s) formées par des microcapsules à libération modifiée du (des) principe(s) actif(s), dont le pH de déclenchement est égal à 6,0 ou 6,5.

28. Médicament selon l'une des revendications 21 à 27, **caractérisé en ce que** le profil de libération, mesuré dans un test de libération in vitro est tel qu'indiqué ci après:
∘ moins de 20 % du principe(s) actif(s) sont libérés au bout de 2 heures à pH = 1,4;
∘ au moins 50 % du principe(s) actif(s) sont libérés au bout de 16 heures à pH = 1,4.

29. Médicament selon l'une des revendications 21 à 28, **caractérisé en ce qu'**il comprend au moins une population de micro-unités contenant du principe(s) actif(s) à libération immédiate, dont le comportement dans un test de dissolution in vitro est tel qu'au moins 80 % du(ou des) principe(s) actif(s) sont libérés en 1 heure à tout pH compris entre 1,4 et 7,4.

30. Médicament selon l'une des revendications 21 à 29, **caractérisé en ce que** la proportion de principe(s) actif(s) peu soluble dans les microunités contenant du principe(s) actif(s) (exprimée en % en poids sur sec par rapport à la masse totale des micro-unités) est comprise entre 5 et 80, de préférence entre 10 et 70, et, plus préférentiellement encore entre 15 et 60.

31. Médicament selon l'une des revendications 21 à 30, **caractérisé en ce que** les micro-unités contenant du(ou des) principe(s) actif(s) à libération immédiate sont des microparticules non enrobées.

32. Médicament selon l'une des revendications précédentes, **caractérisé en ce qu'**il se présente sous forme de dose unique orale journalière comprenant de 5000 à 500000 microcapsules à libération modifiée de principe(s) actif(s).

33. Médicament selon l'une des revendications précédentes, **caractérisé en ce qu'**il se présente sous forme de sachet de poudre de microcapsules, de suspension liquide de microcapsules, de comprimé obtenu à partir de microcapsules, ou de gélule contenant des microcapsules.

34. Médicament selon l'une des revendications précédentes **caractérisé en ce que** le (ou les) principe(s) actif(s) peu(ven)t être choisi(s) parmi au moins l'une des grandes variétés de substances actives, suivantes : antiulcéreux, antidiabétiques, anticoagulants, antithrombiques, hypolipémiants, antiarythmiques, vasodilatateurs, antiangineux, antihypertenseurs, vasoprotecteurs, promoteurs de fécondité, inducteurs et inhibiteurs du travail utérin, contraceptifs, antibiotiques, antifongiques, antiviraux, anticancéreux, anti inflammatoires, analgésiques, antiépileptiques, antiparkinsoniens, neuroleptiques, hypnotiques, anxiolytiques, psychostimulants, antimigraineux, antidepresseurs, antitussifs, antihistaminiques ou antiallergiques, agents permettant de lutter contre l'insuffisance cardiaque congestive, l'angine de poitrine, l'hypertrophie ventriculaire gauche, les arythmies cardiaques, les infarctus du myocarde, la tachycardie réflexe, la maladie cardiaque ischémique, l'athéromatose, l'hypertension associée au diabète mellitus, l'hypertension portale, les vertiges, l'abradicardie, l'hypotension artérielle, la rétention hydrosodée, l'insuffisance rénale aigue, l'hypotension orthostatique et la congestion cérébrale, et leurs mélanges.

35. Médicament selon l'une des revendications précédentes **caractérisé en ce que** le (ou les) principe(s) actif(s) est (sont) choisis dans le groupe de produits comprenant: acide acétylsalycilique, carbamazepine pentoxifylline, prazosine, acyclovir, nifedipine, diltiazem, naproxen, ibuprofen, flurbiprofen, ketoprofen, fenoprofen, indométhacine, diclofenac, fentiazac, oestradiol valérate, métoprolol, sulpiride, captopril, cimetidine, zidovudine, nicardipine, terfenadine, atenolol, salbutamol, carbamazépine, ranitidine, énalapril, simvastatine, fluoxétine, alprazolam, famotidine, ganciclovir, famciclovir, spironolactone, 5-asa, quinidine, périndopril, morphine, pentazocine, paracétamol, oméprazole, lansoprazole, métoclopramide, acide aminosalicylique, acide nalidixique, amoxicilline, amoxicilline et clavulanate de potassium, ampicilline, ampicilline et sulbactame, azithromycine, bacampicilline, carbénicilline-indanyl-sodium (et autres sels de carbénicilline), capréomycine, céfadroxile, céfazoline, céphalexine, céphalothine, céphapirine, céphacelor, céphprozile, céphadrine, céfamandole, céfonicide, céforanide, céfuroxime, céfixime, céfopérazone, céfotaxime, cefpodoxime, ceftaxidime, ceftibuten, ceftizoxime, ceftriaxone, cefepime, cefmétazole, céfotetane, céfoxitine, ciprofloxacine, clarithromycoine, clindamycine, clofazimine, cloxacilline, co-triamoxazole, cyclosérine, dicloxacilline, dirithromycine, érythromycine (et sels d'érythromycine tels que estolate, éthylsuccinate, gluceptate, lactobionate, stéarate), éthambutol-HCI et autres sels, éthionamide, fosfomycine, imipenem, isoniazide, levofloxacine, lomefloxacine, loracarbef, méthicilline, méthenamine, métronidazole, métoclopramide, mezlocilline, nafcilline, nitrofurantoine, norfloxacine, novobiocine, ofloxacine, oxacilline, pénicilline V, sels de pénicilline, complexes de pénicilline, pentamidine, pipéracilline, pipéracilline et tazobactame, sparfloxacine, sulfacytine, sulfamérazine, sulfaméthazine, sulfaméthixole, sulfasalazine, sulfisoxazole, sulfapyrizine, sulfadiazine, sulfméthoxazole, sulfapyridine, ticarcilline, ticarcilline et clavulanate de potassium, triméthoprime, trimétrexate, troléanomycine, vancomycine, vérapamil et leurs mélanges.

36. Médicament selon l'une des revendications précédentes **caractérisé en ce que** le (ou les) principe(s) actif(s) est (sont) un (des) principe(s) actif(s) peu soluble(s).

37. Microcapsules telles que définies dans l'une quelconque des revendications précédentes.

## Patentansprüche

1. Orales Medikament, umfassend eine Vielzahl von Mikrokapseln für eine modifizierte Freisetzung von Wirkstoff bzw. Wirkstoffen, wobei mindestens ein Teil der Mikrokapseln einzeln aus einem Mikropartikel bestehen, das mindestens einen Wirkstoff enthält, insbesondere mindestens einen wenig löslichen Wirkstoff mit Ausnahme von Carvedilol, umhüllt von mindestens einer Beschichtung, die eine modifizierte Freisetzung des Wirkstoffs bzw. der Wirkstoffe gestattet, wobei die Freisetzung durch zwei unterschiedliche Auslösemechanismen gesteuert wird, von denen einer auf einer Veränderung des pH basiert und der andere die Freisetzung des Wirkstoffs bzw. der Wirkstoffe nach einer zuvor festgelegten Verweildauer im Magen gestattet,
wobei die Beschichtung den Mikrokapseln außerdem ein derartiges in vitro-Lösungsverhalten verleiht, dass:
- bei konstantem pH-Wert 1,4 das Lösungsprofil eine Latenzphase mit einer Dauer von weniger als oder gleich 7 Stunden, vorzugsweise weniger als oder gleich 5 Stunden und noch stärker bevorzugt zwischen 1 bis 5 Stunden enthält;
- das Übergehen von pH 1,4 nach pH 7,0 zu einer Freisetzungsphase führt, die ohne Latenzzeit beginnt;
**dadurch gekennzeichnet,**
- **dass** mindestens ein Teil der Mikrokapseln mindestens ein Quellmittel umfasst,
- und dadurch, dass der Gewichtsanteil des Wirkstoffs bzw. der Wirkstoffe, der/die während der Latenzphase freigesetzt wird/werden, weniger als oder gleich 15 Gew.-% pro Stunde, vorzugsweise weniger als oder gleich 10 Gew.-% pro Stunde und noch stärker bevorzugt weniger als oder gleich 5 Gew.-% pro Stunde beträgt.

2. Medikament nach Anspruch 1, **dadurch gekennzeichnet, dass** das Quellmittel mindestens eine pharmazeutisch annehmbare, hydrophile Verbindung umfasst, die ein Quellverhältnis in Wasser bei 25°C von größer als oder gleich 10 Gew.-%, vorzugsweise größer als oder gleich 15 Gew.-% und noch stärker bevorzugt größer als oder gleich 20% aufweist.

3. Medikament nach Anspruch 1, **dadurch gekennzeichnet, dass** das Quellmittel aus denjenigen ausgewählt ist, die es den Mikrokapseln ermöglichen, mindestens 50 Gew.-% des Wirkstoffs nach 16 Std. bei pH 1,4 in einem in-vitro-Lösungstest freizusetzen.

4. Medikament nach Anspruch 1, **dadurch gekennzeichnet, dass** das Quellmittel in Form von Mikropartikeln mit einem mittleren Durchmesser zwischen 5 und 200 µm und vorzugsweise zwischen 10 und 50 µm vorliegt.

5. Medikament nach Anspruch 1, **dadurch gekennzeichnet, dass** die Konzentration (Cd) des Quellmittels zwischen 3 Gew.-% und 40 Gew.-%, bezogen auf die Gesamtmasse der Mikrokapseln, vorzugsweise zwischen 4 Gew.-% und 30 Gew.-% und noch stärker bevorzugt zwischen 5 Gew.-% und 25 Gew.-% beträgt.

6. Medikament nach Anspruch 1, **dadurch gekennzeichnet, dass** das Quellmittel aus der Gruppe der folgenden Produkte ausgewählt ist:
∘ vernetzten Polyvinylpyrrolidonen (zum Beispiel Polyplasdon oder Crospovidon)
∘ vernetzten Carboxyalkylcellulosen: vernetzten Carboxymethylcellulosen (zum Beispiel Croscarmellose)
∘ sowie hydrophilen Polymeren mit hoher Molekülmasse (größer als oder gleich 100.000 D), wie:
▪ Polyvinylpyrrolidone,
▪ Polyalkylenoxide (zum Beispiel Polyethylenoxid oder Polypropylenoxid),
▪ (Hydroxy) (alkyl) Cellulosen (zum Beispiel Hydroxypropylcellulose, Hydroxypropylmethylcellulose),
▪ Carboxyalkylcellulosen (zum Beispiel Carboxymethylcellulose),
▪ Cellulosen (Pulver oder mikrokristallin),
▪ modifizierte Stärken (zum Beispiel mit Natriumglykolat),
▪ natürliche Stärken (zum Beispiel aus Mais, Weizen oder Kartoffeln),
▪ Natriumalginat,
▪ Polacrilin-Kalium
▪ und deren Gemische.

7. Medikament nach einem der vorhergehenden Ansprüche, **dadurch gekennzeichnet, dass** es mindestens ein Benetzungsmittel umfasst, das vorzugsweise aus der Gruppe der folgenden Produkte ausgewählt ist:
∘ anionischen Tensiden, vorzugsweise aus der Untergruppe der Alkali- oder Erdalkalisalze von Fettsäuren, wobei Stearinsäure und/oder Ölsäure bevorzugt sind,
∘ nichtionischen Tensiden, vorzugsweise aus der folgenden Untergruppe:
▪ polyoxyethylenierte Öle, vorzugsweise polyoxyethyleniertes hydriertes Rizinusöl,
▪ Polyoxyethylen-Polyoxypropylen-Copolymere,
▪ polyoxyethylenierte Sorbitanester,
▪ Derivate von polyoxyethyleniertem Rizinusöl,
▪ Stearate, vorzugsweise Calcium-, Magnesium-, Aluminium- oder Zinkstearat,
▪ Stearylfumarate, vorzugsweise Natriumstearylfumarat,
▪ Glycerinbehenat
▪ und deren Gemische.

8. Medikament nach einem der vorhergehenden Ansprüche, **dadurch gekennzeichnet, dass** das Quellmittel in dem Wirkstoffmikropartikel enthalten ist.

9. Medikament nach einem der vorhergehenden Ansprüche, **dadurch gekennzeichnet, dass** das Benetzungsmittel in dem Wirkstoffmikropartikel enthalten ist.

10. Medikament nach einem der vorhergehenden Ansprüche, **dadurch gekennzeichnet, dass** die Mikrokapseln des Wirkstoffs bzw. der Wirkstoffe, die es umfasst, zur Freisetzung von mindestens 80 Gew.-% des Wirkstoffs bzw. der Wirkstoffe nach 12 Std. bei pH = 7,0 in einem in vitro-Lösungstest fähig sind.

11. Medikament nach einem der vorhergehenden Ansprüche, **dadurch gekennzeichnet, dass** mindestens ein Teil der Mikrokapseln für eine modifizierte Freisetzung des Wirkstoffs Folgendes aufweist:
∘ einen neutralen Kern,
∘ mindestens eine aktive Schicht, die den Wirkstoff bzw. die Wirkstoffe umfasst und den neutralen Kern umhüllt, und
∘ mindestens eine Beschichtung, die eine modifizierte Freisetzung des Wirkstoffs gestattet.

12. Medikament nach einem der vorhergehenden Ansprüche, **dadurch gekennzeichnet, dass**:
∘ die Beschichtung, die die modifizierte Freisetzung des Wirkstoffs bzw. der Wirkstoffe gestattet, ein Verbundmaterial umfasst,
▪ umfassend:
• mindestens ein hydrophiles Polymer A, das Gruppen trägt, die bei neutralem pH ionisiert sind,
• mindestens eine hydrophobe Verbindung B;
▪ das einem Massenanteil (Gew.-% bezogen auf die Gesamtmasse der Mikrokapseln) von weniger als oder gleich 40% darstellt;
o die Mikrokapseln einen mittleren Durchmesser von weniger als 2000 µm besitzen.

13. Medikament nach Anspruch 12, **dadurch gekennzeichnet, dass** das Verbundmaterial AB der Beschichtung, das die modifizierte Freisetzung des wenig löslichen Wirkstoffs gestattet, derart ist, dass:
∘ das Gewichtsverhältnis B/A zwischen 0,2 und 1,5, vorzugsweise zwischen 0,5 und 1,0 beträgt,
∘ und die hydrophobe Verbindung B aus kristallinen Produkten im festen Zustand ausgewählt ist, die eine Schmelztemperatur SchmB von mehr als oder gleich 40°C, vorzugsweise SchmB von mehr als oder gleich 50°C und noch stärker bevorzugt zwischen 40°C und 90°C besitzen.

14. Medikament nach einem der Ansprüche 12 und 13, **dadurch gekennzeichnet, dass** das hydrophile Polymer A aus: Polymeren von (Meth)Acrylsäure, Polymeren von Alkyl (meth) acrylat und Copolymeren von (Meth)Acrylsäure und Alkyl(meth)acrylat, Cellulosederivaten, Cellulosephthalaten, Celluloseacetatphthalat, Cellulosesuccinaten, Celluloseacetatsuccinat, Hydroxypropylmethylcellulosephthalaten, Hydroxypropylmethylcellulosesuccinaten und deren Gemischen ausgewählt ist.

15. Medikament nach einem der Ansprüche 12 bis 14, **dadurch gekennzeichnet, dass** die Verbindung B aus der Gruppe der folgenden Produkte ausgewählt ist: hydrierten Pflanzenölen, Pflanzenwachsen, gelbem Bienenwachs, weißem Wachs, mikrokristallinem Wachs, Lanolin, wasserfreien Milchfettstoffen, Hartfett oder Fett, das als Grundlagen für Suppositorien geeignet ist, Laurylmakrogolglyceriden, Cetylalkohol, Polyglycerindiisostearat, Monoestern, Diestern und Triestern von Glycerin und mindestens einer Fettsäure, Gemischen von Monoestern, Diestern und Triestern von Glycerin und mindestens einer Fettsäure, Ethylenglykolmonostearat, Diethylenglykolmonostearat und deren Gemischen.

16. Medikament nach Anspruch 15, **dadurch gekennzeichnet, dass** die Verbindung B aus der Gruppe der folgenden Produkte ausgewählt ist: hydriertem Baumwollsamenöl, hydriertem Sojabohnenöl, hydriertem Palmöl, Glycerinbehenat, hydriertem Rizinusöl, Tristearin, Tripalmitin, Trimyristin, Omega 3 und jedem beliebigen Gemisch von diesen.

17. Medikament nach einem der Ansprüche 12 bis 16, **dadurch gekennzeichnet, dass** die Beschichtung der Mikrokapseln für eine modifizierte Freisetzung des Wirkstoffs einen einzigen Überzugsfilm umfasst, der den Verbundstoff AB enthält.

18. Orales Medikament, umfassend eine Vielzahl von Mikrokapseln für eine modifizierte Freisetzung von Wirkstoff bzw. Wirkstoffen, wobei mindestens ein Teil der Mikrokapseln einzeln aus einem Mikropartikel besteht, das mindestens einen Wirkstoff umfasst, insbesondere mindestens einen wenig löslichen Wirkstoff mit Ausnahme von Carvedilol, umhüllt von mindestens einer Beschichtung, die eine modifizierte Freisetzung des Wirkstoffs bzw. der Wirkstoffe gestattet, wobei die Freisetzung durch zwei unterschiedliche Auslösemechanismen gesteuert wird, von denen einer auf einer Veränderung des pH basiert und der andere die Freisetzung des Wirkstoffs bzw. der Wirkstoffe nach einer zuvor festgelegten Verweildauer im Magen gestattet,
wobei die Beschichtung:
∘ den Mikrokapseln außerdem ein derartiges in vitro-Lösungsverhalten verleiht, dass:
- bei konstantem pH-Wert 1,4 das Lösungsprofil eine Latenzphase mit einer Dauer von weniger als oder gleich 7 Stunden, vorzugsweise weniger als oder gleich 5 Stunden und noch stärker bevorzugt zwischen 1 bis 5 Stunden enthält;
- das Übergehen von pH 1,4 nach pH 7,0 zu einer Freisetzungsphase führt, die ohne Latenzzeit beginnt;
∘ und ein Verbundmaterial umfasst, das mindestens ein hydrophiles Polymer A, das Gruppen trägt, die bei neutralem pH ionisiert sind, und mindestens eine hydrophobe Verbindung B enthält;
**dadurch gekennzeichnet, dass** mindestens ein Teil der Mikrokapseln mindestens ein Freisetzungshilfsmittel umfasst, welches die Permeabilität der Beschichtung erhöhen kann, was eine modifizierte Freisetzung des Wirkstoffs bzw. der Wirkstoffe gestattet, und dadurch, dass der Gewichtsanteil des Wirkstoffs bzw. der Wirkstoffe, der/die während der Latenzphase freigesetzt wird/werden, weniger als oder gleich 15 Gew.-% pro Stunde, vorzugsweise weniger als oder gleich 10 Gew.-% pro Stunde und noch stärker bevorzugt weniger als oder gleich 5 Gew.-% pro Stunde beträgt.

19. Medikament nach Anspruch 18, **dadurch gekennzeichnet, dass** das Freisetzungshilfsmittel aus mindestens einem Quellmittel besteht.

20. Medikament nach Anspruch 18 oder 19, **dadurch gekennzeichnet, dass** die Beschichtung der Mikrokapseln diesen ein derartiges in vitro-Lösungsverhalten verleiht, dass mindestens 50 Gew.-% des Wirkstoffs bzw. der Wirkstoffe nach 16 Std. bei pH 1,4 freigesetzt sind.

21. Medikament insbesondere, aber nicht beschränkend nach einem der Ansprüche 1 bis 20, **dadurch gekennzeichnet, dass** es ein Gemisch von verschiedenen Populationen von Mikroeinheiten umfasst, die den Wirkstoff bzw. die Wirkstoffe mit Ausnahme von Carvedilol enthalten, wobei sich diese Populationen voneinander durch ihre jeweiligen in vitro-Lösungsprofile für mindestens einen pH-Wert zwischen 1,4 und 7,4 unterscheiden.

22. Medikament nach Anspruch 20 und nach mindestens einem der anderen vorhergehenden Ansprüche, **dadurch gekennzeichnet, dass** es sich bei den Mikroeinheiten um Mikrokapseln für eine modifizierte Freisetzung des Wirkstoffs bzw. der Wirkstoffe und gegebenenfalls um Mikroeinheiten für eine sofortige Freisetzung des Wirkstoffs bzw. der Wirkstoffe handelt.

23. Medikament nach Anspruch 21, **dadurch gekennzeichnet, dass** die Populationen von Mikrokapseln für eine modifizierte Freisetzung des Wirkstoffs sich anhand ihrer jeweiligen Auslöse-pH-Werte unterscheiden.

24. Medikament nach Anspruch 21, **dadurch gekennzeichnet, dass** die Populationen von Mikrokapseln für eine modifizierte Freisetzung des Wirkstoffs sich anhand ihrer jeweiligen Auslösungszeiten unterscheiden.

25. Medikament nach Anspruch 21, **dadurch gekennzeichnet, dass** es Folgendes umfasst:
i. mindestens eine Population von Mikroeinheiten, die Wirkstoff für eine sofortige Freisetzung enthält;
ii. mindestens eine Population P1 von Mikrokapseln für eine modifizierte Freisetzung des Wirkstoffs bzw. der Wirkstoffe und
iii. mindestens eine Population P2 von Mikrokapseln für eine modifizierte Freisetzung des Wirkstoffs bzw. der Wirkstoffe; und dadurch, dass die jeweiligen Auslöse-pH-Werte von P1 und P2 sich um mindestens 0,5 pH-Einheiten, vorzugsweise um mindestens 0,8 pH-Einheiten und noch stärker bevorzugt um mindestens 0,9 pH-Einheiten unterscheiden.

26. Medikament nach Anspruch 21, **dadurch gekennzeichnet, dass** die jeweiligen Auslöse-pH-Werte der verschiedenen Populationen von Mikrokapseln für eine modifizierte Freisetzung des Wirkstoffs bzw. der Wirkstoffe zwischen 5 und 7 betragen.

27. Medikament nach Anspruch 21, **dadurch gekennzeichnet, dass** es Folgendes umfasst:
i. mindestens eine Population von Mikroeinheiten, die Wirkstoff bzw. Wirkstoffe für eine sofortige Freisetzung enthalten,
ii. mindestens eine Population P1' von Mikroeinheiten, die Wirkstoff bzw. Wirkstoffe enthalten, die durch Mikrokapseln für eine modifizierte Freisetzung des Wirkstoffs bzw. der Wirkstoffe gebildet werden, deren Auslöse-pH gleich 5,5 ist, und
iii. mindestens eine Population P2' von Mikroeinheiten, die Wirkstoff bzw. Wirkstoffe enthalten, die durch Mikrokapseln für eine modifizierte Freisetzung des Wirkstoffs bzw. der Wirkstoffe gebildet werden, deren Auslöse-pH gleich 6,0 oder 6,5 ist.

28. Medikament nach einem der Ansprüche 21 bis 27, **dadurch gekennzeichnet, dass** das in einem in vitro-Freisetzungstest gemessene Freisetzungsprofil wie im Folgenden angegeben ist:
∘ weniger als 20% des Wirkstoffs bzw. der Wirkstoffe sind nach 2 Stunden bei pH = 1,4 freigesetzt;
∘ mindestens 50% des Wirkstoffs bzw. der Wirkstoffe sind nach 16 Stunden bei pH = 1,4 freigesetzt.

29. Medikament nach einem der Ansprüche 21 bis 28, **dadurch gekennzeichnet, dass** es mindestens eine Population von Mikroeinheiten umfasst, die Wirkstoff bzw. Wirkstoffe für eine sofortige Freisetzung enthalten, deren Verhalten in einem in vitro-Lösungstest derart ist, dass mindestens 80% des Wirkstoffs bzw. der Wirkstoffe in 1 Std. bei jedem pH-Wert zwischen 1,4 und 7,4 freigesetzt sind.

30. Medikament nach einem der Ansprüche 21 bis 29, **dadurch gekennzeichnet, dass** der Anteil des wenig löslichen Wirkstoffs bzw. der wenig löslichen Wirkstoffe in den Mikroeinheiten, die den Wirkstoff bzw. die Wirkstoffe enthalten, (ausgedrückt in Trockengewichts-% bezogen auf die Gesamtmasse der Mikroeinheiten) zwischen 5 und 80, vorzugsweise zwischen 10 und 70 und noch stärker bevorzugt zwischen 15 und 60 beträgt.

31. Medikament nach einem der Ansprüche 21 bis 30, **dadurch gekennzeichnet, dass** die Mikroeinheiten, die den Wirkstoff bzw. die Wirkstoffe für eine sofortige Freisetzung enthalten, nicht-umhüllte Mikropartikel sind.

32. Medikament nach einem der vorhergehenden Ansprüche, **dadurch gekennzeichnet, dass** es in Form einer täglichen oralen Einzeldosis, umfassend 5000-500 000 Mikroeinheiten für eine modifizierte Freisetzung des Wirkstoffs bzw. der Wirkstoffe, vorliegt.

33. Medikament nach einem der vorhergehenden Ansprüche, **dadurch gekennzeichnet, dass** es in Form eines Beutels mit einem Mikrokapselpulver, einer flüssigen Suspension von Mikrokapseln, einer aus den Mikrokapseln erhaltenen Tablette oder einer die Mikrokapseln enthaltenden Gelatinekapsel vorliegt.

34. Medikament nach einem der vorhergehenden Ansprüche, **dadurch gekennzeichnet, dass** der Wirkstoff bzw. die Wirkstoffe aus mindestens einem der großen Vielfalt der folgenden Wirkstoffe ausgewählt werden kann/können: Antigeschwürmitteln, Antidiabetika, Antikoagulantien, Antithrombotika, Lipidsenkern, Antiarrhythmika, Vasodilatatoren, Antianginamittel, Antihypertensiva, Vasoprotektoren, Fruchtbarkeitsförderern, Induktoren und Inhibitoren der Uterusaktivität, Kontrazeptiva, Antibiotika, Antipilzmitteln, antiviralen Mitteln, Antikrebsmitteln, entzündungshemmenden Mitteln, Analgetika, Antiepileptika, Anti-Parkinson-Mitteln, Neuroleptika, Hypnotika, Anxiolytika, Psychostimulanzien, Antimigränemitteln, Antidepressiva, Antitussiva, Antihistaminika oder Antiallergika, Mitteln zur Bekämpfung von kongestivem Herzversagen, Angina pectoris, linksventrikulärer Hypertrophie, Herzrhythmusstörungen, Myokardinfarkt, reflektorischer Tachykardie, ischämischer Herzkrankheit, Atheromatose, durch Diabetes mellitus bedingter Hypertonie, portaler Hypertonie, Schwindel, Abradykardie, arterieller Hypotonie, Wasser- und Natriumretention, akuter Niereninsuffizienz, orthostatischer Hypotonie und Hirninfarkt, und deren Gemischen.

35. Medikament nach einem der vorhergehenden Ansprüche, **dadurch gekennzeichnet, dass** der Wirkstoff bzw. die Wirkstoffe aus der Gruppe von Produkten ausgewählt ist (sind), umfassend: Acetylsalicylsäure, Carbamazepin Pentoxifyllin, Prazosin, Acyclovir, Nifedipin, Diltiazem, Naproxen, Ibuprofen, Flurbiprofen, Ketoprofen, Fenoprofen, Indometacin, Diclofenac, Fentiazac, Östradiolvalerat, Metoprolol, Sulpirid, Captopril, Cimetidin, Zidovudin, Nicardipin, Terfenadin, Atenolol, Salbutamol, Carbamazepin, Ranitidin, Enalapril, Simvastatin, Fluoxetin, Alprazolam, Famotidin, Ganciclovir, Famciclovir, Spironolacton, 5-ASA, Chinidin, Perindopril, Morphin, Pentazocin, Paracetamol, Omeprazol, Lansoprazol, Metoclopramid, Aminosalicylsäure, Nalidixinsäure, Amoxicillin, Amoxicillin und Kaliumclavulanat, Ampicillin, Ampicillin und Sulbactam, Azithromycin, Bacampicillin, Carbenicillin-Indanyl-Natrium (und andere Carbenicillinsalze), Capreomycin, Cefadroxil, Cefazolin, Cephalexin, Cephalothin, Cephapirin, Cephacelor, Cephprozil, Cephadrin, Cefamandol, Cefonicid, Ceforanid, Cefuroxim, Cefixim, Cefoperazon, Cefotaxim, Cefpodoxim, Ceftaxidim, Ceftibuten, Ceftizoxim, Ceftriaxon, Cefepim, Cefmetazol, Cefotetan, Cefoxitin, Ciprofloxacin, Clarithromycin, Clindamycin, Clofazimin, Cloxacillin, Co-Triamoxazol, Cycloserin, Dicloxacillin, Dirithromycin, Erythromycin (und Erythromycinsalze, wie Estolat, Ethylsuccinat, Gluceptat, Lactobionat, Stearat), Ethambutol-HCl und andere Salze, Ethionamid, Fosfomycin, Imipenem, Isoniazid, Levofloxacin, Lomefloxacin, Loracarbef, Methicillin, Methenamin, Metronidazol, Metoclopramid, Mezlocillin, Nafcillin, Nitrofurantoin, Norfloxacin, Novobiocin, Ofloxacin, Oxacillin, Penicillin V, Salze von Penicillin, Komplexe von Penicillin, Pentamidin, Piperacillin, Piperacillin und Tazobactam, Sparfloxacin, Sulfacytin, Sulfamerazin, Sulfamethazin, Sulfamethixol, Sulfasalazin, Sulfisoxazol, Sulfapyrizin, Sulfadiazin, Sulfmethoxazol, Sulfapyridin, Ticarcillin, Ticarcillin und Kaliumclavulanat, Trimethoprim, Trimetrexat, Troleanomycin, Vancomycin, Verapamil und deren Gemischen.

36. Medikament nach einem der vorhergehenden Ansprüche, **dadurch gekennzeichnet, dass** es sich bei dem Wirkstoff bzw. den Wirkstoffen um einen wenig löslichen Wirkstoff bzw. wenig lösliche Wirkstoffe handelt.

37. Mikrokapseln nach einem der vorhergehenden Ansprüche.

## Claims

1. Oral medicinal product comprising a plurality of microcapsules with modified release of active principle(s), at least some of said microcapsules individually consisting of a microparticle comprising at least one active principle, in particular at least one active principle with low solubility, with the exclusion of carvedilol, coated with at least one coating for the modified release of the active principle(s), said release being controlled by two distinct triggering mechanisms, one being based on a variation in pH and the other allowing the release of the active principle(s) after a predetermined time of residence in the stomach,
said coating also conferring on the microcapsules an in vitro dissolution behaviour such that:
- at constant pH 1.4, the dissolution profile comprises a lag phase of less than or equal to 7 hours, preferably less than or equal to 5 hours, and even more preferably of between 1 and 5 hours;
- the transition from pH 1.4 to pH 7.0 results in a release phase that begins without any lag time;
**characterized**
**in that** at least some of said microcapsules comprise at least one swelling agent,
and **in that** the weight fraction of the active principle(s) released during the lag phase is less than or equal to 15% by weight per hour, preferably less than or equal to 10% by weight per hour, and even more preferably less than or equal to 5% by weight per hour.

2. Medicinal product according to claim 1, **characterized in that** the swelling agent comprises at least one hydrophilic pharmaceutically acceptable compound exhibiting a degree of swelling in water at 25°C of greater than or equal to 10% by weight, preferably greater than or equal to 15% by weight, and even more preferably greater than or equal to 20%.

3. Medicinal product according to claim 1, **characterized in that** the swelling agent is chosen from those that allow the microcapsules to release at least 50% by weight of the active principle after 16 h at pH 1.4, in an in vitro dissolution test.

4. Medicinal product according to claim 1, **characterized in that** the swelling agent is in form of microparticles with a mean diameter between 5 and 200 µm, and preferably between 10 and 50 µm.

5. Medicinal product according to claim 1, **characterized in that** the concentration (Cd) of swelling agent is between 3% and 40% by weight relative to the total weight of the microcapsules, preferably between 4% and 30% and even more preferably between 5% and 25%.

6. Medicinal product according to claim 1, **characterized in that** the swelling agent is chosen from the group of following products:
∘ crosslinked polyvinylpyrrolidones (e.g. polyplasdone or crospovidone),
∘ crosslinked carboxyalkylcelluloses: crosslinked carboxymethylcelluloses (e.g. croscarmellose),
∘ and also hydrophilic polymers with high molar weights (greater than or equal to 100 000 D), such as:
▪ polyvinylpyrrolidones,
▪ polyalkylene oxides (e.g. polyethylene oxide or polypropylene oxide),
▪ (hydroxy) (alkyl) celluloses (e.g. hydroxypropylcellulose, hydroxypropylmethylcellulose),
▪ carboxyalkylcelluloses (e.g. carboxymethylcellulose),
▪ celluloses (powder or microcrystalline),
▪ modified starches (for example modified with sodium glycolate),
▪ natural starches (for example cornstarch, wheat starch or potato starch),
▪ sodium alginate,
▪ polacrilin potassium,
▪ and mixtures thereof.

7. Medicinal product according to one of the preceding claims, **characterized in that** it comprises at least one wetting agent, preferably chosen from the group of following products:
• anionic surfactants, preferably from the subgroup of alkali metal salts or alkaline-earth metal salts of fatty acids, stearic acid and oleic acid being preferred,
• nonionic surfactants, preferably from the following subgroup:
∘ polyoxyethylenated oils, preferably hydrogenated polyoxyethylenated castor oil,
∘ polyoxyethylene-polyoxypropylene copolymers,
∘ polyoxyethylenated sorbitan esters,
∘ polyoxyethylenated castor oil derivatives,
∘ stearates, preferably calcium stearate, magnesium stearate, aluminium stearate or zinc stearate,
∘ stearylfumarates, preferably sodium stearylfumarate,
∘ glyceryl behenate,
∘ and mixtures thereof.

8. Medicinal product according to one of the preceding claims, **characterized in that** the swelling agent is contained in the microparticle of active principle.

9. Medicinal product according to one of the preceding claims, **characterized in that** the wetting agent is contained in the microparticle of active principle.

10. Medicinal product according to one of the preceding claims, **characterized in that** the microcapsules of active principle(s) that it comprises release at least 80% by weight of the active principle(s) after 12 h at pH = 7.0, in an in vitro dissolution test.

11. Medicinal product according to one of the preceding claims, **characterized in that** at least some of said microcapsules with modified release of active principle comprise:
∘ a neutral core,
∘ at least one active layer comprising the active principle(s) and coating the neutral core, and
∘ at least one coating for modified release of the active principle.

12. Medicinal product according to one of the preceding claims, **characterized in that**:
∘ the coating for modified release of the active principle(s) comprises a composite material
▪ comprising:
• at least one hydrophilic polymer A carrying groups that are ionized at neutral pH,
• at least one hydrophobic compound B;
▪ representing a weight fraction (% weight relative to the total wright of the microcapsules) less than or equal to 40%;
∘ the microcapsules have a mean diameter of less than 2000 µm.

13. Medicinal product according to claim 12, **characterized in that** the composite material AB of the coating for modified release of the active principle with low solubility, is such that:
▪ the B/A weight ratio is between 0.2 and 1.5, preferably between 0.5 and 1.0,
▪ and the hydrophobic compound B is selected from products that are crystalline in the solid state and that have a melting point MpB greater than or equal to 40°C, preferably MpB greater than or equal to 50°C, and even more preferably between 40°C and 90°C.

14. Medicinal product according to either of claims 12 and 13, **characterized in that** the hydrophilic polymer A is chosen from: polymers of (meth)acrylic acid polymers , alkyl(meth)acrylate polymers, copolymers of (meth)acrylic acid and of alkyl(meth)acrylate, cellulose derivatives, cellulose phthalates, cellulose acetate phthalate, cellulose succinates, cellulose acetate succinate, hydroxypropyl methylcellulose phthalates, hydroxypropyl methylcellulose succinates, and mixtures thereof.

15. Medicinal product according to one of claims 12 to 14, **characterized in that** the compound B is chosen from the group of following products: hydrogenated vegetable oils, vegetable waxes, yellow wax, monocrystalline wax, lanolin, anhydrous milk fat, hard fat or fat that is useful as suppository bases, lauryl macrogolglycerides, cetyl alcohol, polyglyceryl diisostearate, diesters and triesters of glycerol with at least one fatty acid, mixtures of monoesters, diesters and triesters of glycerol with at least one fatty acid, ethylene glycol monostearate, diethylene glycol monostearate, and mixtures thereof.

16. Medicinal product according to claim 15, **characterized in that** the compound B is chosen from the group of following products: hydrogenated cottonseed oil, hydrogenated soybean seed oil, hydrogenated palm oil, glyceryl behenate, hydrogenated castor oil, tristearin, tripalmitin, trimyristin, trimyristin, Omega 3 and any mixture thereof.

17. Medicinal product according to any one of claims 12 to 16, **characterized in that** the coating of the microcapsules with modified release of active principle comprises a single coating film comprising the composite AB.

18. Oral medicinal product comprising a plurality of microcapsules with modified release of active principle(s), at least some of said microcapsules individually consisting of a microparticle comprising at least one active principle, in particular at least one active principle with low solubility, with the exclusion of carvedilol, coated with at least one coating for modified release of the active principle(s), said release being controlled by means of two distinct triggering mechanisms, one based on a variation in pH and the other allowing release of the active principle(s) after a predetermined residence time in the stomach,
said coating:
▪ also conferring on the microcapsules an in vitro dissolution behaviour such that:
- at constant pH 1.4, the dissolution profile comprises a lag phase of less than or equal to 7 hours, preferably less than or equal to 5 hours, and even more preferably of between 1 and 5 hours;
- the transition from pH 1.4 to pH 7.0 results in a release phase that begins without any lag time;
▪ and comprising a composite material comprising at least one hydrophilic polymer A carrying groups that are ionized at neutral pH and at least one hydrophobic compound B;
**characterized in that** at least some of said microcapsules comprise at least one release helper for increasing the permeability of the coating for modified release of the active principle(s),
and **in that** the weight fraction of the active principle(s) released during the lag phase is less than or equal to 15% by weight per hour, preferably less than or equal to 10% by weight per hour, and even more preferably less than or equal to 5% by weight per hour.

19. Medicinal product according to claim 18, **characterized in that** the release helper consists of at least one swelling agent.

20. Medicinal product according to claim 18 or 19, **characterized in that** the coating of the microcapsules confers on them an in vitro dissolution behaviour such that at least 50% by weight of the active principle(s) are released after 16 h at pH 1.4.

21. Medicinal product, in particular but without limitation according to one of claims 1 to 20, **characterized in that** it comprises a mixture of various populations of microunits containing active principle(s) with the exclusion of carvedilol, these populations differing from one another through their respective in vitro dissolution profiles, for at least one pH value of between 1.4 and 7.4.

22. Medicinal product according to claim 20 and at least one of the other preceding claims, **characterized in that** the micro-units are microcapsules with modified release of active principle(s) and, optionally, micro-units with immediate release of active principle(s).

23. Medicinal product according to claim 21, **characterized in that** the populations of microcapsules with modified release of active principle differ through their respective triggering pHs.

24. Medicinal product according to claim 21, **characterized in that** the populations of microcapsules with modified release of active principle differ through their respective triggering times.

25. Medicinal product according to claim 21, **characterized in that** it comprises:
i. at least one population of micro-units containing active principle with immediate release;
ii. at least one population P1 of microcapsules with modified release of active principle(s); and
iii. at least one population P2 of microcapsules with modified release of active principle(s);
and **in that** the respective triggering pHs of P1 and of P2 differ by at least 0.5 pH unit, preferably by at least 0.8 pH unit, and even more preferably by at least 0.9 pH unit.

26. Medicinal product according to claim 21, **characterized in that** the respective triggering pHs of the various populations of microcapsules with modified release of active principle(s) are between 5 and 7.

27. Medicinal product according to claim 21, **characterized in that** it comprises:
i. at least one population of micro-units containing active principle(s) with immediate release;
ii. at least one population P1' of micro-units containing active principle(s), formed by microcapsules with modified release of the active principle(s), for which the triggering pH is equal to 5.5; and
iii. at least one population P2' of micro-units containing active principle(s), formed by microcapsules with modified release of the active principle(s), for which the triggering pH is equal to 6.0 or 6.5.

28. Medicinal product according to one of claims 21 to 27, **characterized in that** the release profile, measured in an in vitro release test, is as indicated below:
• less than 20% of the active principle(s) are released after 2 hours at pH = 1.4;
• at least 50% of the active principle(s) are released after 16 hours at pH = 1.4.

29. Medicinal product according to one of claims 21 to 28, **characterized in that** it comprises at least one population of micro-units containing active principle(s) with immediate release, the behaviour of which in an in vitro dissolution test is such that at least 80% of the active principle(s) is released in 1 hour at any pH between 1.4 and 7.4.

30. Medicinal product according to one of claims 21 to 29, **characterized in that** the proportion of low soluble active principle(s) in the micro-units containing active principle(s) (expressed as % by weight on a dry basis relative to the total weight of the micro-units) is between 5 and 80, preferably between 10 and 70, and even more preferably between 15 and 60.

31. Medicinal product according to one of Claims 21 to 30, **characterized in that** the micro-units containing active principle(s) with immediate release are non-coated microparticles.

32. Medicinal product according to one of the preceding claims, **characterized in that** it is provided in the form of a single daily oral dose comprising from 5000 to 500 000 microcapsules with modified release of active principle(s).

33. Medicinal product according to one of the preceding claims, **characterized in that** it is provided in form of a sachet of microcapsules powder, a liquid suspension of microcapsules, a tablet obtained from microcapsules, or a gelatin capsule containing microcapsules.

34. Medicinal product according to one of the preceding claims, **characterized in that** the active principle(s) may be chosen from at least one of the following major varieties of active substances: antiulcer agents, antidiabetic agents, anticoagulants, antithrombics, blood lipidlowering agents, antiarythmics, vasodilators, anti-angina agents, antihypertensives, vasoprotective agents, fertility promoters, inducers and inhibitors of uterine labour, contraceptives, antibiotics, antifungal agents, antiviral agents, anticancer agents, anti-inflammatories, analgesics, antiepileptics, antiParkinsonian agents, neuroleptics, hypnotics, anxiolytics, psychostimulants, antimigraine agents, antidepressants, antitussives, antihistamines or antiallergic agents, agents for combating congestive heart failure, angina pectoris, left ventricular hypertrophy, cardiac arythmias, myocardial infarcttions, reflex tachycardia, ischaemic heart disease, atheromatosis, hypertension related to diabetes mellitus, portal hypertension, dizziness, bradycardia, arterial hypotension, hydrosodic retention, acute kidney failure, orthostatic hypotension and cerebral congestion, and mixtures thereof.

35. Medicinal product according to one of the preceding claims, **characterized in that** the active principle(s) is (are) chosen from the group of products comprising: acetylsalicylic acid, carbamazepine, pentoxifylline, prazosine, acyclovir, nifedipine, diltiazem, naproxen, ibuprofen, flurbiprofen, ketoprofen, fenoprofen, indomethacin, diclofenac, fentiazac, oestradiol valerate, metoprolol, sulpiride, captopril, cimetidine, zidovudine, nicardipine, terfenadine, atenolol, salbutamol, carbamazepine, ranitidine, enalapril, simvastatin, fluoxetine, alprazolam, famotidine, ganciclovir, famciclovir, spironolactone, 5-asa, quinidine, perindopril, morphine, pentazocine, paracetamol, omeprazole, lansoprazole, metoclopramide, aminosalicylic acid, nalidixic acid, amoxicillin, amoxicillin and clavulanate potassium, ampicillin, ampicillin and sulbactam, azithromycin, bacampicillin, carbenicillin indanyl sodium (and other carbenicillin salts), capreomycin, cefadroxil, cefazolin, cephalexin, cephalothin, cephapirin, cephacelor, cefprozil, cephadrine, cefamandole, cefonicide, ceforanide, cefuroxime, cefixime, cefoperazone, cefotaxime, cefpodoxime, ceftaxidime, ceftibuten, ceftizoxime, ceftriaxone, cefepime, cefmetazole, cefotetan, cefoxitin, ciprofloxacine, clarithromycin, clindamycin, clofazimine, cloxacillin, cotriamoxazole, cycloserine, dicloxacillin, dirithromycin, erythromycin (and erythromycin salts such as estolate, ethylsuccinate, gluceptate, lactobionate, stearate), ethambutol-HCl and other salts, ethionamide, fosfomycin, imipenem, isoniazide, levofloxacine, lomefloxacine, loracarbef, methicillin, methenamine, metronidazole, metoclopramide, mezlocillin, nafcillin, nitrofurantoin, norfloxacin, novobiocin, ofloxacin, oxacillin, penicillin V, penicillin salts, penicillin complexes, pentamidine, piperacillin, piperacillin and tazobactam, sparfloxacin, sulphacytine, sulphamerazine, sulphamethazine, sulphamethixole, sulphasalazine, sulphisoxazole, sulphapyrizine, sulphadiazine, sulphmethoxazole, sulphapyridine, ticarcillin, ticarcillin and clavulanate potassium, trimethoprime, trimetrexate, troleanomycin, vancomycin, verapamil and mixtures thereof.

36. Medicinal product according to one of the preceding claims, **characterized in that** the active principle(s) is (are) one of the active principle(s) with low solubility.

37. Microcapsules as defined in any one of the preceding claims.
